(19) Europäisches Patentamt

European Patent Office

Office européen des brevets

(11) **EP 3 912 622 A1**

(12) **EUROPEAN PATENT APPLICATION**

(43) Date of publication:
24.11.2021 Bulletin 2021/47

(51) Int Cl.:
**A61K 31/05** (2006.01)   **A61K 9/00** (2006.01)
**A61K 38/28** (2006.01)   **A61P 25/06** (2006.01)

(21) Application number: 20175523.8

(22) Date of filing: 19.05.2020

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **tesa Labtec GmbH
40764 Langenfeld (DE)**

(72) Inventors:
• **Lubenow, Helge
40764 Langenfeld (DE)**

• **Niese, Svenja
40597 Düsseldorf (DE)**
• **Braun, Sebastian
42929 Wermelskirchen (DE)**
• **Ernst, Steffen
435 37 Mölnlycke (SE)**
• **Lutz, Eva Saskia Mareike
43169 Mölndal (SE)**

(74) Representative: **Hemsath, Lars et al
König-Szynka-Tilmann-von Renesse
Patentanwälte Partnerschaft mbB
Mönchenwerther Straße 11
40545 Düsseldorf (DE)**

(54) **ORAL FILM FOR SAFE ADMINISTRATION OF API**

(57) The present invention relates to dosage units of oral films for the administration of active pharmaceutical ingredients (API), such as analgesics, relaxants, anxiolytics, sedatives, hypnotics, narcotics, anesthetics and/or other API with peripheral and/or central nervous effects, in particular for use in the treatment of pain, metabolic disorders and diseases of the central nervous system (CNS). More particular the invention relates to dosage units of oral films comprising relaxants, anxiolytics, sedatives, hypnotics, narcotics, anesthetics and/or analgesics prone to misuse and abuse, such as overdosing. More particular, the invention relates to dosage units of oral films comprising propofol.

In a further aspect, the invention relates to dosage units of oral films comprising propofol for use in the treatment of a subject suffering from migraine.

EP 3 912 622 A1

**Description**

**FIELD OF THE INVENTION**

The present invention relates to dosage units of oral films for the administration of active pharmaceutical ingredients (API), such as analgesics, relaxants, anxiolytics, sedatives, hypnotics, narcotics, anesthetics and/or other API with peripheral and/or central nervous effects, in particular for use in the treatment of pain, metabolic disorders and diseases of the central nervous system (CNS). More particular the invention relates to dosage units of oral films comprising relaxants, anxiolytics, sedatives, hypnotics, narcotics, anesthetics and/or analgesics prone to misuse and abuse, such as overdosing. More particular, the invention relates to dosage units of oral films comprising propofol.

In a further aspect, the invention relates to dosage units of oral films comprising propofol for use in the treatment of a subject suffering from migraine.

**BACKGROUND OF THE INVENTION**

Active pharmaceutical ingredients (API) such as analgesics, anesthetics, narcotics, sedatives, hypnotics, anxiolytics or hormones that exert a potent therapeutic effect and are liable to misuse, abuse, the generation of addictions and in particular overdosing, wherefore such API are usually administered in a clinical setting or by a trained medical practitioner. These API are frequently administered, also in combination, in the treatment and management of diseases including metabolic disorders, diseases of the nervous system, pain and combinations thereof. To achieve a systemic effect, these API are often administered by injection or infusion. Another route of systemic administration for API is oral administration, which is however not suited for every API as the milieu of the oral cavity or the gastrointestinal tract can inactivate the API or resorption into blood circulation can be insufficient or occur with too much time delay.

Likewise, API administered in the context of metabolic disorders or CNS diseases include macromolecules, such as hormones and enzymes. These API can be liable to misuse, abuse, the generation of addiction and overdosing. Therefore, these API are frequently administered in a clinical setting or after introduction by a trained medical practitioner.

Dosage forms of a drug are the physical characteristics of that drug product, such as a tablet, a capsule or a solution. The various dosage forms are suited for certain administration routes. The dosage form contains the API and other ingredients such as excipients, fillers, flavors, preservatives, emulsifiers etc. Orally disintegrating films (ODF) and oral films with mucoadhesive properties are considered an alternative dosage form from classical dosage forms. They are different from well established dosage forms such as lozenges, effervescent tablets, powders, granules for the production of a solution or other solid pharmaceutical preparations for oral administration. ODF and oral films with mucoadhesive properties can be applied for local delivery or systemic resorption of the comprised API. Parts of the API are usually resorbed over the mucosa in the oral cavity and other parts of the API are resorbed with a delay in time after swallowing of the API from the gastrointestinal tract. ODF are frequently used to rapidly provide an API, particularly to rapidly provide an API to the CNS.

Overdosing of potent API such as analgesics, anesthetics, narcotics, sedatives, hypnotics and/or hormones, which can be potentially lethal, is frequently done by injection. Hormones such as insulin or glycoproteins such as interferon beta are regularly administered by injection. Moreover, oral dosage forms including those with retard formulations are frequently crushed and subsequently injected by a misuser or abuser. Dosage forms for alternative administration routes render such endevours more difficult. However, misuse, abuse, illicit use and overdosing also occur via alternative routes of administration. Abuse is commonly considered as the intentional, permanent or sporadic overuse of medicines with physical or psychological damage as a result thereof (c.f. RL 2011/83/EG or 16th amendment of the Medicinal Products Act). In the context of drug abuse, the API is consumed with the intention to be under the influence of the drug for pleasure or commiting suicide. While abuse is generally done intentionally, misuse, the development of addictions and overdosing can occur intentionally or unintentionally. For example, the overdosing of paracetamol is the most frequent cause of acute liver failure in Europe and the US and currently responsible for more cases of acute liver failure than all other etiologies combined (Jaeschke H. Acetaminophen: Dose-Dependent Drug Hepatotoxicity and Acute Liver Failure in Patients. Dig Dis 2015;33:464-471; Ghanem C.I., Perez M.J., Manautou J.E., Mottino A.D., Maria J.P. Acetaminophen from liver to brain: New insights into drug pharmacological action and toxicity. Pharm. Res. 2016;109:119-131. doi: 10.1016/j.phrs.2016.02.020). Such overdosing of paracetamol often occurs unintentionally. Therefore, many API suffer from dosage forms and dosage units that enable their administration in doses at which they exert a harmful or toxic effect.

This applies even more to API which are administered to children. Children are generally more sensitive with regard to the selected dose of an API and even a slight increase in the administered dose can cause a harmful or toxic effect in children. Hence, there is a need to limit the delivery of potential harmful API in non-human and human patients and in particular in children to prevent a toxic effect. In particular, there is a need to limit the dose applicable with a single or multitude of a dosage unit.

A different approach for delivery of an API such as an analgesic and/or anesthetic is through oral or mucosal application of API comprising dosage forms. WO 2011/061332 describes a composition comprising propofol as liquid formulation or gel that may be administered via the oral or nasal mucosa or by inhalation. Moreover, dissolving films comprising as API analgesics, anesthetics, narcotics or sedatives, such as triptans for oral application (WO 2010/062688 A1 or WO 2018/091473 A1) or lidocaine for nasal application (WO 2005/055977 A2), are known in the art. Likewise, dosage units of dosage forms comprising hormones that are films for oral, buccal, sublingual or transdermal administration are known (WO 2018/208701 A1).

Hence, one approach for systemic delivery of an API such as an analgesic, anesthetic, narcotic, sedative and/or hypnotic commonly administered is through orally dissolvable thin film strips. US 2011/0269844 A1 describes an orally dissolvable thin film strip for administration of propofol that may be edible or for transdermal delivery of propofol. The propofol released from according edible film strips may be resorbed in the blood stream.

However, a rapid delivery of large quantities of an API which can exert a harmful or toxic effect when administered in an excessive dose facilitates abuse and misuse of the active substance, the development of addictions and the potential of overdosing. Therefore, there is a need in the art for an improved dosage unit that prevents abuse and misuse of the comprised API and enables administration of non-toxic doses by technically limiting the abuse and misuse of said dosage forms.

Hence, it is an objective of the present invention to provide a dosage unit of an oral film comprising an API which can exert a harmful or toxic effect when administered in a dose that is higher than a therapeutic dose of the respective API such as an analgesic, anesthetic, narcotic, sedative, hypnotic and/or hormone which prevents the administration of doses of the respective API that exert a toxic effect. Moreover, it is an objective of the present invention to provide a dosage unit of an oral film comprising an API that technically prevents that a certain dose of the API can be exceeded if one or a multitude of said dosage unit are applied simultaneously or within a certain time frame after each other.

## SUMMARY OF THE INVENTION

This objective is solved with the dosage unit of a mucoadhesive oral film according to claim 1, namely by a dosage unit comprising an API wherein the dosage of said API is limited in such a way that in case of covering all non-keratinized parts of the mouth of the subject by multiple said dosage units, the API cannot be administered in a dose at which the API has a toxic effect.

The inventors started from the realization that the application area in the mouth from where a transmucosal delivery into systemic circulation can be achieved in the oral cavity is limited. The inventors have further found that the effective systemic dose set by transmucosal delivery of an API that can have a toxic effect such as an analgesic, anesthetic, narcotic, sedative, hypnotic and/or hormone can be prevented to exceed the therapeutic dose of said API and/or to exert a toxic effect by a dosage unit of an oral mucoadhesive thin film which provides a technical limiting combination of a limited application area and/or limited dose of the API prevents abuse and misuse such as overdosing. Moreover, the inventors have found that according dosage units can comprise doses of potent API such as analgesics and/or anesthetics that do not exceed or are even much lower than analgesic, anesthetic, narcotic, sedative and/or hypnotic therapeutic doses, i.e. that the dosage units according to the invention may deliver a dose of the API comprised that is for instance lower than that required and/or subtherapeutic for an analgesic, anesthetic, narcotic, sedative and/or hypnotic therapeutic effect, but which enables the comprised API to exert other desirable therapeutic effects at these lower and/or subtherapeutic dose levels. The therapeutic effects of an API at lower or subtherapeutic dose levels can occur via a different mechanism than the effect at analgesic, anesthetic, narcotic, sedative or hypnotic therapeutic dose levels, whereby the dose delivered is subtherapeutic with regard to an analgesic, anesthetic, narcotic, sedative and/or hypnotic effect. Delivery of lower and/or subtherapeutic doses may result from the technical limitation to the API dosage according to claim 1 of the invention and/or from means affecting the transmucosal and/or transbuccal delivery over non-keratinized mucosal parts in the mouth.

The solution according to the invention prevents abuse and misuse of the API comprised in the dosage unit such as overdosing and prevents administration of dosages for which the occurrence of adverse events can be readily expected. Hence, the invention relates to dosage units of oral mucoadhesive films comprising API which can exert a toxic effect when applied in a dosage that is higher than that recommended for a safe and efficacious risk-benefit balance or API which do not have a safe risk-benefit balance. Such API can be selected from API such as analgesics, anesthetics, narcotics, sedatives, hypnotics or hormones for which the total applied dose that can be delivered by a multitude of respective dosage units which would cover all non-keratinized parts of the mouth is limited by a combination of a limited application area and a limited dose of the API thereby technically limiting their combined API dosage, the total deliverable dose of said API.

Furthermore, the invention relates to the medical use of a dosage units in the treatment of subjects suffering from pain, in particular a migraine pain. The invention also relates to the medical use of said dosage units in the treatment of subjects suffering from a neurological disease, such as a disease of the central nervous system, such as migraine, in particular a migraine without pain. Moreover, the invention relates to the medical use of the dosage units in the treatment of subjects suffering from a headache, in particular a primary headache, more particular the treatment of subjects suffering from a migraine, most particular a migraine with or without pain. Advantageously, the technical limitation of the API dosage deliverably by a dosage unit according to the invention and the prevention of abuse and/or misuse enables the prescription-free or prescription-dependent, over-the-counter distribution of the respective dosage units, which enables an unsupervised self-administration, an acute use and/or an outpatient use.

The inventors realized that the mouth of a full-grown adult human subject has a surface area of about 220 cm$^2$, whereof about 50%, i.e. about 110 cm$^2$ are non-keratinized parts, so-called non-keratinized oral mucosa over which an API for entry into systemic circulation can be administered. Likewise, the inventors realized that the non-keratinized surface area of the mucosa of the mouth of a not full-grown human subject is limited. The non-keratinized surface area of the mucosa of the mouth of a not full-grown human subject usually does not exceed that in the mouth of a full-grown human subject. Usually the non-keratinized surface area of the mucosa of the mouth of a not full-grown human subject approximately ranges between 45 to 110 cm$^2$. Newborn not full-grown human subjects commonly have a non-keratinized surface area of the mucosa of the mouth of about 45 cm$^2$. This area increases as the not full-grown human subject ages and develops further into a full-grown human subject.

Therefore, the inventors realized that the application area for API comprising films in the oral cavity is limited and could be used to technically limit the delivered amount of API, in particular API prone to abuse and misuse, such as overdosing. The dosage unit according to the invention can be brought in direct contact with this non-keratinized oral mucosa by mucoadhesion ensuring timely systemic delivery. By limiting the combined dosage of API that can be delivered by one or many said dosage units according to the invention via the entire non-keratinized oral mucosa, through a combination of limited area and limited API dose per dosage unit, it is prevented that a dosage is applied which exerts a toxic effect. Therefore, the administration of respective API is rendered safe and abuse and misuse are prevented.

The API which are most suitable for use in the present invention are API that can be delivered very well by mucosa uptake, and have a low oral bioavailability when adsorbed from the gastrointestinal tract. Preferably the API are inactivated after swallowing. Suitable API can have a rapid onset of action, a potent or highly potent effect, a short plasma half-life or a relatively low cumulation. Such API may belong to the group of analgesics that are be applied in step 2 and step 3 of the WHO pain ladder group and/or the group of anesthetics, narcotics, sedatives or hypnotics that allow for a mild to deep sedation. Such API can be very liable to abuse, misuse or overdosing. Typically, such API can have fatal consequences when overdosed. Further API very suitable for use in the present invention belong to the group of hormones, in particular proteohormones, more particular bioidentical hormones. Hormones are frequently misused for example in the context as performance enhancing drugs. Thus, hormones are API that can be readily overdosed. Mild to deep sedation can be classified as a score of $\leq$ -2 on the Richmond Agitation and Sedation Scale (RASS). One example of such an API is the sedative propofol (2,6-diisopropylphenol or propofolum), which is an API from the group of anesthetics, more particular from the group of sedatives. Other preferred API for use in the present invention are anesthetics, opioids, muscle relexants, benzodiazepine, etomidiat, barbiturates and hormones, the most prefered API of the invention is propofol.

The dosage form most suitable for use in the present invention is that of an oral film. Oral films render endevours to extract the comprised API for abuse and misuse difficult. They often comprise the API in a low concentration, the dosage form may not be easy to dissolve and it is difficult and/or uneconomical to extract the API.

This principle is exemplified with propofol ($C_{12}H_{18}O$). Propofol belongs to the group of anesthetics, within the group of anesthetics, it is classified as a sedative hypnotic API. Propofol is the INN, the ACD/IUPAC term for propofol is 2,6-diisopropylphenol. The ATC-code is AX10 and the CAS number 2078-54-8. Currently available dosage forms are exclusively for injection, these dosage forms are usually concentrated at 10 mg/ml in puncture bottles or available in single use vials for parenteral applications. A 1% solution or emulsion of propofol is a solution or emulsion containing 10 mg of propofol per 1 ml, likewise a 2% solution propofol contains 20 mg propofol per ml. Propofol is commercially available for example under the trade names Disoprivan®, Diprivan® or Recofol® as well as generics. It is believed to work at least partially via a receptor for gamma-Aminobutyric acid (GABA).

Current intravenous administration schemes for onset of anesthesia for 1% solutions of Propofol, such as the SMPC of Diprivan®, recommend that in unpremedicated and premedicated patients the 1% solution of propofol should be titrated against the response of the patient until the clinical signs show the onset of anesthesia. The required dose for induction of anesthesia is in most adult patients aged less than 55 years usually between 1.5 - 2.5 mg/kg body weight of the 1% solution of propofol. In patients over this age, the requirement is usually less. The recommended dose requirement for older patients for induction of anesthesia using a 1% propofol solution is reduced compared to patients aged less than 55 years. Use of 1% solution of propofol in children aged less than 1 month is not recommended. Recommendations for the dose requirement for induction of anesthesia in children over 1 month are that a 1% solution of propofol should be titrated slowly until clinical signs shown the onset of anesthesia. The required dose for induction of anesthesia using a 1 % solution of propofol in children over 8 years is usually about 2.5 mg/kg body weight. The required dose for induction of anesthesia using a 1 % solution of propofol in children aged 1 month to 8 years is usually between 2.5 to 4 mg/kg body weight. Alternatively, a therapeutic dose of propofol should not exceed 4 mg/kg body weight/h.

With regard to the minimum effective concentration of propofol that is required for induction of an anesthetic therapeutic effect of propofol, an average blood concentration of 4.05 $\pm$ 1.01 $\mu$g/ml is required for major surgeries, whereas 2.97 $\pm$ 1.07 $\mu$g /ml levels are sufficient for minor surgical procedures. A lower blood level of 1.5 - 2.0 $\mu$g/ml is required for the sedative effect of propofol (Dhir et al., 2016). To prevent an unwanted toxic effect of propofol, an average blood concentration of 4.05 $\pm$ 1.01 $\mu$g/ml must be prevented. After onset of sedation propofol requires continuous dosing by intravenous injection and infusion to maintain the sedative effect. The required rate of administration varies considerably between patients. Usually a rate between 4-12 mg/kg body weight/h maintains the anesthetic effect in adults under the age of 55. In children over the age of 1 month rates in the region of 9 - 15 mg/kg body weight/h maintain the anesthesia. Alternatively, in children over the age of 1 month anesthesia can be maintained by repeated bolus injection. In younger children, the dose requirement for maintenance of anesthesia can be higher than 9-15 mg/kg body weight/h.

In an embodiment of the invention, one or multiple dosage units according to the invention prevent that an effective systemic concentration of propofol of at least 1.5 - 2.0 $\mu$g/ml can be reached. In a different embodiment of the invention, one or multiple dosage units according to the invention prevent that an effective systemic concentration of propofol of 4.05 $\pm$ 1.01 $\mu$g/ml can be reached. In a different embodiment of the invention, one or multipe dosage units according to the invention prevent that a dose of at least 1.5 mg/kg body weight propofol can be reached, in particular prevent that a dose of at least 1.5 mg/kg body weight propofol can be reached in a full-grown subject. In a different embodiment of the invention, one or multiple dosage units according to the invention prevent that a dose of at least 2.5 mg/kg body weight propofol can be reached, in particular prevent that a dose of at least 2.5 mg/kg body weight propofol can be reached in a not full-grown subject.

The current administration regimen for propofol is administration by intravenous (i.v.) injection and infusion. Recommendations for induction of onset of sedation are hypnotic doses of propofol of 1.5 (minimum) to 2.5 (maximum) mg/kg bodyweight, amounting to a minimum dose of 105.0 mg of propofol for induction of sedation and a maximum dose of 175.0 mg for a subject of 70 kg. Hence, for a subject of 70 kg a therapeutic sedative dose of propofol can also be considered for the range of 105.0 to 175.0 mg, a therapeutic subsedative dose propofol can be considered for a dose below 105 mg/kg body weight. In order to not exceed the sedative therapeutic dose for a subject of 70 kg, the dosage provided by one or many dosage units when combined to cover the non-keratinized parts of the mouth should not exceed 105 - 175.0 mg. In a different embodiment, a subsedative dose of propofol can be considered for a effective systemic concentration of less than 1.5 - 2.0 $\mu$g/ml.

Hence, for a dosage unit according to the present invention the dose propofol could be selected as for example not more than 10.0 mg as maximum dose for a dosage unit. In a next step, the area of the dosage unit is selected. This selection is performed such that the area of a dosage unit when combined with further said dosage units to a combined area of about the area of the non-keratinized mucosal parts in the mouth for transmucosal delivery, which in full-grown humans cover an area of about 110 cm$^2$ and can cover less surface area, such as 45 to 110 cm$^2$, in a non full-grown human, limits the combined API dose, i.e. the total API dose applied to the mucosa, to not more than a dose that can be safely applied to induce a therapeutic effect and/or prevent a toxic effect. In other words, when a certain propofol dose per dosage unit is selected, the surface area for application of the dosage unit onto a non-keratinized part in the mouth is selected accordingly, i.e. maximized, to technically limit the ability to apply multiple said films to the extend that a hypnotic effect is exceeded and a toxic effect can occur. Hence, the area of an according dosage unit comprising 10.0 mg propofol can be selected from an area of at least about 6.3 cm$^2$ in order to limit the combined API dose at 175.0 mg, as not more than 17 of said dosage unit can be placed onto the non-keratinized parts of the oral mucosa simultaneously. Hence, 17 of said dosage units with a dose of 10 mg propofol can deliver at most 170 mg propofol and the safe and efficient dosage limit of 175.0 mg propofol cannot be exceeded and an unwanted toxic effect is prevented. Accordingly, if the API dose per dosage unit is reduced this minimum area is also reduced. Similarly, if the dose of propofol must not exceed 105.0 mg as otherwise an unwanted toxic effect could occur, the area of a dosage unit comprising 10.0 mg propofol can be selected from an area of at least about 10.5 cm$^2$. Advantageously, for propofol, the total dose applied when selecting the dose of the dosage unit that in case of covering all non-keratinized parts of the mouth of said subject by multiple said dosage units remains below the dose for a sedative effect, enabling further, therapeutic effects of propofol, with different modes of action than that for the hypnotic effect.

Alternatively, the area of the dosage unit can be selected first and the dosage of the API can be determined in a second step, whereby the combined dosage of one or many essentially equal dosage units to a combined area that equals the area of the non-keratinized mucosal parts of the mouth in a full-grown adult, i.e. an area of about 110 cm$^2$, which can be less in a non full-grown human, is limited to prevent that a toxic effect is exerted by the API. Exemplified for propofol, the area of the dosage unit can be selected to be 6 cm$^2$ with a maximum of 175.0 mg propofol suitable for a therapeutic sedative effect, i.e. at a higher combined dose of propofol it might exert a toxic effect. Accordingly the dosage of the dosage unit could be selected to be not more than about 9.5 mg. Likewise, if the maximum of propofol suitable for a sedative therapeutic effect was 105.0 mg, for a dosage unit of 6 cm$^2$, the dosage of the dosage unit could be selected to be not more than about 5.7 mg.

In a preferred aspect of the invention the API is selected as an anesthetic, preferably a sedative hypnotic, even more preferably as propofol and the area a dosage unit comprising propofol is selected as at least 6 cm$^2$.

In a different preferred aspect of the invention, the API is selected as an anesthetic, preferably a sedative hypnotic, even more preferably propofol and the dosage of the dosage unit is selected as less than 5 mg per dosage unit and the area of the dosage unit is selected as at least 6 cm$^2$.

In a different aspect the dosage unit of the invention, the API comprised in the dosage unit of the invention can be selected from different group of API.

In a further aspect the dosage unit is selected for the treatment of a particular disease. A dosage unit according to the invention can be applied in different diseases.

Moreover, the inventors have realized that mucoadhesive dosage units of oral films can increase and maximize the quantities of an API comprised in the dosage unit that are resorbed over the mucosa. Hence, in a further aspect, the general principle according to the invention is especially advantageous when the residence time of the dosage unit, i.e. the time during which the dosage unit is in contact with the non-keratinized parts of the mouth, is limited. Residence time can for instance be affected by the time of mucoadhesion and/or disintegration time of the dosage unit. The residence time may be used to limit the delivery time of the API comprised in the dosage units. Advantageously, in this aspect applying several of said dosage units one after the other has no additional effect on the effective systemic level of the API, as the delivery time of the API can be limited to a time to essentially equal the time for flattening of the systemic level of the API or the delivery time of the API can be limited to exceed the time for flattening of the systemic level of the API via the residence time. In a particularly preferred aspect, delivery time of the API corresponds in essence to the residence time of the dosage unit.

In a further aspect of the invention, the residence time can be selected in relation to the half-life, in particular the systemic half-life, of the API comprised in the dosage unit. Advantageously the residence time is selected about equal or longer to the half-life of API. In this aspect, the systemic level of the API declines at about the same rate or faster than the API can be administered using the dosage unit. Preferably, in this aspect, the API is selected as an analgesic, anesthetic, narcotic, hypnotic and/or sedative.

In yet another aspect of the invention dosage unit comprising propofol can be used for the treatment of migraine, whereby the migraine is optionally selected from migraine with aura with pain, migraine with aura without pain, such as acephalgic migraine, ocular migraine or retinal migraine, migraine without aura, chronic migraine, pediatric migraine, menstrual migraine, intractable migraine, refractory migraine or acute confusional migraine (ACM).

In yet another aspect, the invention relates to compositions of dosage units. Favorable, pleasant organoleptic features increase the likelihood of abuse and misuse of an API. Accordingly, compositions of the dosage unit do not mask the taste of the dosage unit. Preferably, to this end the compositions are essentially free of flavoring agents and/or sweetening agents. According compositions comprise an API, a solvent, a polymer and optionally a plasticizer.

Other objects, features, advantages and aspects of the present invention will become apparent to those skilled in the art from the following description and appended claims.

It should be understood, however, that the following description, appended claims and specific examples, which indicate preferred embodiments of the application, are given by way of illustration only. Various changes and modifications within the essence and scope of the disclosed invention will become readily apparent to those skilled in the art form reading the following.

## DETAILED DESCRIPTION OF THE INVENTION

As used herein, the expressions defined herein are generally intended to preferably have the meanings as set forth below, except to the extent that the context in which they are used indicates otherwise.

The percentage values defined herein in general refer to weight-per-weight percentage values unless otherwise indicated. In preferred embodiments, the values given herein encompass a range of $\pm$ 5 %, preferably of $\pm$ 2 % or $\pm$ 1 %.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

The term "dosage unit" as used in the context of this invention refers to a pharmaceutical product of a particular dose. In the context of this invention a dosage unit can be used to deliver at least one API. A dosage unit refers to a unit of a pharmaceutical product with a particular dosage. A dosage unit can refer to different dosage forms such as dosage forms for oral, nasal, dermal, ophthalmic, parenteral, topical, suppository or inhalation administration. A dosage unit can also refer to solid, liquid or gaseous pharmaceutical products. Preferably a dosage unit refers to a pharmaceutical product for nasal or oral administration, most preferably for oral administration. Preferably a dosage unit refers to a solid pharmaceutical product. In particular a dosage unit refers to a pharmaceutical product, e.g. an oral film of a particular dose and area. Advantageously, the dosage unit according to the invention prevents a toxic effect, such as an effect resulting from abuse and misuse including overdosing and/or the administration of a potentially lethal dose of a respective API and allows prescription-free, over-counter distribution of the respective dosage unit, the acute use and/or an outpatient application of a respective API in an according dosage unit becomes possible.

A "dosage" as used in the context of this invention refers to a selected dose of an API comprised. The selected dose of an API comprised in the dosage unit is selected that a toxic effect is prevented irrespective if one or multiple of said dosage unit are applied to the non-keratinized area of the mucosa in the mouth. Moreover, a dosage unit can have an application area, an area over of contact the dosage unit with the non-keratinized mucosa over which the API can be delivered. In a preferred embodiment, the dosage unit according to the present invention is a dosage unit of a mucoadhesive oral film. Advantageously mucoadhesion enables a maintained direct contact between the dosage unit and the mucosa, improving the systemic delivery of the API comprised in the dosage unit. Accordingly, mucoadhesive dosage units of oral films increase and maximize the ratios of the API comprised in the dosage unit that are resorbed over the mucosa. Therefore, the systemic delivery of API that are inactivated during their presence in the gastrointestinal tract and/or oral cavity can be improved. Typically, the dose of the API comprised in a dosage unit is selected first, before the area. Alternatively, the area of the dosage unit can be selected first, before the dose is selected. In an embodiment, the dose of the API of the dosage unit is selected in combination with the area of the dosage unit and the residence time of the dosage unit. A dosage unit can be referred to as a combination of a dose of an API and an area of the dosage unit. A dosage unit can therefore limit the amount of API that is applicable over a certain application area. Advantageously, a dosage unit comprises an API in a dose selected from a range pre-defined in relation to therapeutic doses of the comprised API in such way that in case all, theoretically available, non-keratinized parts of the mouth of the subject are covered by said dosage units, a toxic effect of the API comprised is prevented.

In a preferred embodiment, API are selected for a dosage unit according to the invention which are inactivated in the gastrointestinal tract. In this embodiment, the API is resorbed essentially via the non-keratinized mucosa in the mouth and absorbed systemically so as to bypass the first-pass metabolism.

The dose of an API comprised in a dosage unit is selected in combination with an area of the dosage unit. The dose can be selected first and the area is selected depending on the selected dose of the API in the dosage unit or the area is selected first and the dose is selected depending on the selected area of the dosage unit. In an embodiment the dose is selected first. In a different embodiment, the area is selected first. In a further different embodiment, the dose of the API comprised and the area of the dosage unit are selected simultaneously. Selection of the dose of the API per dosage unit is performed based on the therapeutic dose of the selected API, whereby a therapeutic dose is a dose which is recommended according to a safe and efficacious risk-benefit balance or in cases in which a risk-benefit balance is absent a dose known to the skilled person to be safe and efficacious. If the selected API comprises an analgesic, the dose of the analgesic is selected as not more than a maximal dose for a therapeutic effect as analgesic, i.e. a dose at which the API exerts an analgesic function and not a toxic effect. If the selected API comprises an anesthetic, the dose of the anesthetic is selected as not more than a maximal dose for a therapeutic effect as anesthetic, i.e. a dose at which the API exerts a sedative, hypnotic and/or anesthetic effect and not a toxic effect. If the selected API comprises a hormone, the dose of the hormone is selected as not more than a maximal dose for a therapeutic effect as hormone, i.e. a dose at which the API exerts a therapeutic effect as hormone and does not have a toxic effect.

In an embodiment in which an analgesic is selected as API, the dose of the analgesic per dosage unit can be not more than 200 mg, not more than 150 mg, not more than 100 mg, not more than 80 mg, not more than 50 mg, not more than 10 mg, not more than 5 mg, about 5 mg, less than 5 mg, 1 to 2.5 mg, 2.5 to 5 mg, 5 to 10 mg, 10 to 15 mg or 15 to 20 mg of the selected analgesic. Preferably, the dose of the analgesic is selected as less than 5 mg, 1 to 2.5 mg or 2.5 to 5 mg.

In an embodiment in which an anesthetic is selected as API, the dose of the anesthetic per dosage unit can be about 5 mg, less than 5 mg, 1 to 2.5 mg, 2.5 to 5 mg, 5 to 10 mg, 10 to 15 mg or 15 to 20 mg of the selected anesthetic. Preferably, the dose of the anesthetic is selected as less than 5 mg, 1 to 2.5 mg or 2.5 to 5 mg.

In an embodiment in which a sedative is selected as API, the dose of the sedative per dosage unit can be about 5 mg, less than 5 mg, 1 to 2.5 mg, 2.5 to 5 mg, 5 to 10 mg, 10 to 15 mg or 15 to 20 mg of the selected sedative. Preferably, the dose of the sedative is selected as less than 5 mg, 1 to 2.5 mg or 2.5 to 5 mg.

In an embodiment in which a hypnotic is selected as API, the dose of the hypnotic per dosage unit can be about 5 mg, less than 5 mg, 1 to 2.5 mg, 2.5 to 5 mg, 5 to 10 mg, 10 to 15 mg or 15 to 20 mg of the selected hypnotic. Preferably, the dose of the hypnotic is selected as less than 5 mg, 1 to 2.5 mg or 2.5 to 5 mg.

In an embodiment in which an analgesic and an anesthetic are selected as API, the dose of the anesthetic per dosage unit can be about 5 mg, less than 5 mg, 1 to 2.5 mg, 2.5 to 5 mg, 5 to 10 mg, 10 to 15 mg or 15 to 20 mg of the selected anesthetic and the dose of the analgesic per dosage unit can be about 5 mg, less than 5 mg, 1 to 2.5 mg, 2.5 to 5 mg, 5 to 10 mg, 10 to 15 mg or 15 to 20 mg of the selected analgesic. Preferably, the dose of the analgesic is selected as about 5 mg, less than 5 mg, 1 to 2.5 mg or 2.5 to 5 mg and the dose of the anesthetic is selected as about 5 mg, less than 5 mg, 1 to 2.5 mg or 2.5 to 5 mg.

In an embodiment in which propofol is selected as API, the dose propofol per dosage unit can be about 5 mg, less than 5 mg, 1 to 2.5 mg, 2.5 to 5 mg, 5 to 10 mg, 10 to 15 mg or 15 to 20 mg of propofol. Preferably, the dose propofol is selected as less than 5 mg, 1 to 2.5 mg or 2.5 to 5 mg.

In an embodiment in which a hormone is selected as API, the dose of the hormone per dosage unit can be about 5 mg, less than 5 mg, 5-10 mg, 10 - 15 mg or 15 - 20 mg of the hormone. In a different embodiment in which a hormone is selected as API the dose of the hormone per dosage unit can be less than 100 IU, 100 - 500 IU, 500 - 1000 IU or 1000 - 2000 IU.

In a preferred embodiment, a dosage unit is a dosage unit of an oral film comprising an API whereby the API is selected as an analgesic and/or an anesthetic.

In another preferred embodiment, a dosage unit is a dosage unit of an oral film for use in the treatment of pain, whereby the pain is preferably selected from headache pain, more preferably a primary headaches, even more preferably from migraines, most preferably from migraines with pain, comprising an anesthetic as API, whereby the anesthetic is selected from the group of anti-depressants, neuroleptics, narcotics, sedatives or hypnotics, preferably selected from sedatives and most preferably selected as propofol.

In another preferred embodiment, a dosage unit is a dosage unit of an oral film for use in the treatment of a neurological disease, such as a disease of the central nervous system, preferably primary headaches, more preferably migraine, most preferably a migraine without pain, comprising an anesthetic as API, whereby the anesthetic is selected from the group of anti-depressants, neuroleptics, narcotics, sedatives or hypnotics, preferably selected from sedatives and most preferably selected as propofol.

In another preferred embodiment, a dosage unit is a dosage unit of an oral film for use in the treatment of migraine comprising an anesthetic as API, whereby the anesthetic is selected from the group of anti-depressants, neuroleptics, anesthetics, narcotics, sedatives or hypnotics, preferably selected from sedatives and hypnotics and more preferably selected as propofol.

The term "area" as used in the context of this invention can refer to an essentially planar expansion. The term can also refer to the application area of a dosage unit that is the area of contact with a mucosa, preferably a mucosa in the mouth, even more preferably all non-keratinized parts of the mucosa in the mouth. The term "area" as used in the context of this invention, can also refer to an area measure, the area the dosage unit extends in a first and a second dimension. In a dosage unit, the area of a dosage unit can be combined with a dose of the API to limit the dose of the API applicable. The area can be selected first and the dose is selected depending on the selected area of the dosage unit or the dose is selected first and the area is selected depending on the selected dose of the dosage unit.

The term "toxic effect" as used in the context of this invention can refer to an applied dose at which the selected API according to a risk-benefit balance does not exert an efficacious and safe therapeutic function. A "toxic effect" can refer to an applied dose at which the sum of the side effects outweighs a potential benefit of the API selected. A "toxic effect" can refer to an applied dose at which the likelihood of adverse effects increases and application would not be considered safe by a skilled person. A "toxic effect" can refer to a dose of an API for which no risk-benefit balance is available but the selected dose is higher than a dose that would be selected by a skilled person as maximum therapeutic dose in a phase II clinical studies or clinical dose finding studies. A "toxic effect" can refer to a selected dose of an API for which no therapeutic effect is available, but the selected dose is known to cause serious, in particular fatal, harmful or toxic effects. A "toxic effect" can also refer to the effect that is achieved by a plasma concentration of an API which is above a therapeutic plasma concentration whereby the increased plasma concentration is a consequence of a certain dose of the API applied. For example, according to a risk-benefit balancing the therapeutic dose of propofol is to be selected as 1.5 - 2.5 mg/kg body weight, with a maximum dose of 4 mg/kg over an hour. Accordingly, at a dose higher than 4 mg/kg the skilled person can expect that the application of propofol will exert a toxic effect. In a different example, the daily insulin requirement in diabetes mellitus type 1 is generally between 0.5 to 1 IU/kg body weight per day in cases of severe insulin resistance > 2 IU/kg body weight per day or 200 IU per day may be required. However, much higher daily doses than 200 IU of insulin should be avoided. Accordingly, at a dose of more than 200 IU of insulin the skilled person can expect that the application of insulin will exert, i.e. have a toxic effect.

Preferred combinations of dose and area according to the present invention, when the dose of the API comprised per dosage unit is selected first, can be derived from the subsequent Table 1. The target combined API dose refers to the approximate dose of the selected API not to be exceeded if multiple of said dosage unit are combined to cover all non-keratinized parts of a mouth. The target combined API dose can be the maximum dose of an API used by the skilled person to induce a respective therapeutic effect according to a risk-benefit balance, accordingly a toxic effect is thereby prevented.

Alternatively, in a different embodiment, the target combined API dose of multiple of said dosage unit is restricted to remain below a minimum dose of an API used by the skilled person to induce a primary therapeutic effect, enabling a secondary therapeutic effect. In this embodiment, exceeding the dose for the secondary therapeutic effect usually does not result in a toxic effect but in the primary therapeutic effect. The primary and the secondary effect that occur at different doses can result from different working mechanisms. The occurrence of the secondary therapeutic effect can be enabled since the dose applied and/or the plasma concentration of the API has fallen and/or remains below the dose and/or plasma concentration required for the primary effect. In this embodiment the total dose of API appliable by a multitude of said dosage units is restricted to remain in the subtherapeutic range of a primary effect, but not to exceed the therapeutic effect of a secondary effect of said API. For instance, a dosage unit according to this embodiment comprising propofol can be restricted in such a manner that if a multitude of said dosage units are applied to cover all non-keratinized parts of the mouth the total dose propofol applied remains below a minimum dose to achieve a sedative or hypnotic effect, i.e. primary therapeutic effect, and not to exceed a dose to achieve an anti-migraine effect, i.e. a secondary therapeutic effect.

In a preferred embodiment the area of the non-keratinized parts of the mouth of a subject, preferably a full-grown human subject, is approximately 110 $cm^2$. Likewise this area is about 45 - 110 $cm^2$ for a not full-grown human subject. The approximate minimum area to be covered by a dosage unit according to the invention can be calculated by dividing the target combined API dose (D) by the selected dose of the selected API per dosage unit (d) in a first step. In a second step, the approximate area of the non-keratinized parts of the mouth (A), which is in a preferred embodiment 110 $cm^2$, is divided by the value of the quotient x obtained in the first step yielding the approximate minimum area to be covered by a single dosage unit (a). In a preferred embodiment, the subject is a full-grown human, the API comprised is propofol and the target combined API dose not to be exceeded to prevent a toxic effect is 175 mg.

This calculation can be visualized as:

Step 1 (Selection of d):

$$D / d = x$$

Step 2 (determination of a):

$$A / x = a$$

With

D: value of the target combined API dose [mg]
d: selected dose of the API comprised in a single dosage unit [mg]
x: quotient of the target combined API dose divided by the selected dose
A: area of the non-keratinized parts of the mouth, in a preferred embodiment this is 110$cm^2$ for a human subject [$cm^2$]
a: approximate minimum area to be covered by the dosage unit for the respected selected API dose [$cm^2$]

Table 1: Preferred combinations of selected dose and area covered per single dosage unit, when the dose of the API is selected first for different target combined API doses.

| Number of combination | Selected API dose per single dosage unit [mg] | Approximate minimum area to be covered by a single dosage unit [$cm^2$] | Target combined API dose [mg] |
|---|---|---|---|
| 1 | 20 | 11.0 | |
| 2 | 19 | 10.5 | |
| 3 | 18 | 9.9 | |
| 4 | 17 | 9.4 | |
| 5 | 16 | 8.8 | |
| 6 | 15 | 8.3 | |
| 7 | 14 | 7.7 | |

(continued)

| Number of combination | Selected API dose per single dosage unit [mg] | Approximate minimum area to be covered by a single dosage unit [cm$^2$] | Target combined API dose [mg] |
|---|---|---|---|
| 8 | 13 | 7.2 | 200 mg or less |
| 9 | 12 | 6.6 | |
| 10 | 11 | 6.1 | |
| 11 | 10 | 5.5 | |
| 12 | 9 | 5.0 | |
| 13 | 8 | 4.4 | |
| 14 | 7 | 4.0 | |
| 15 | 6 | 3.3 | |
| 16 | 5 | 2.8 | |
| 17 | 4 | 2.2 | |
| 18 | 3 | 1.7 | |
| 19 | 2 | 1.1 | |
| 20 | 1 | 0.6 | |
| 21 | 20 | 12.6 | 175 mg or less |
| 22 | 19 | 11.9 | |
| 23 | 18 | 11.3 | |
| 24 | 17 | 10.7 | |
| 25 | 16 | 10.1 | |
| 26 | 15 | 9.4 | |
| 27 | 14 | 8.8 | |
| 28 | 13 | 8.2 | |
| 29 | 12 | 7.5 | |
| 30 | 11 | 6.9 | |
| 31 | 10 | 6.3 | |
| 32 | 9 | 5.7 | |
| 33 | 8 | 5.0 | |
| 34 | 7 | 4.4 | |
| 35 | 6 | 3.7 | |
| 36 | 5 | 3.1 | |
| 37 | 4 | 2.5 | |
| 38 | 3 | 1.9 | |
| 39 | 2 | 1.3 | |
| 40 | 1 | 0.6 | |

(continued)

| Number of combination | Selected API dose per single dosage unit [mg] | Approximate minimum area to be covered by a single dosage unit [cm$^2$] | Target combined API dose [mg] |
|---|---|---|---|
| 41 | 20 | 14.7 | |
| 42 | 19 | 13.9 | |
| 43 | 18 | 13.2 | |
| 44 | 17 | 12.5 | |
| 45 | 16 | 11.7 | |
| 46 | 15 | 11.0 | |
| 47 | 14 | 10.3 | 150 mg or less |
| 48 | 13 | 9.5 | |
| 49 | 12 | 8.8 | |
| 50 | 11 | 8.1 | |
| 51 | 10 | 7.3 | |
| 52 | 9 | 6.6 | |
| 53 | 8 | 5.9 | |
| 54 | 7 | 5.1 | |
| 55 | 6 | 4.4 | |
| 56 | 5 | 3.7 | |
| 57 | 4 | 2.9 | |
| 58 | 3 | 2.2 | |
| 59 | 2 | 1.5 | |
| 60 | 1 | 0.7 | |

(continued)

| Number of combination | Selected API dose per single dosage unit [mg] | Approximate minimum area to be covered by a single dosage unit [cm$^2$] | Target combined API dose [mg] |
|---|---|---|---|
| 61 | 20 | 17.6 | 125 mg or less |
| 62 | 19 | 16.7 | |
| 63 | 18 | 15.8 | |
| 64 | 17 | 15 | |
| 65 | 16 | 14.1 | |
| 66 | 15 | 13.2 | |
| 67 | 14 | 12.3 | |
| 68 | 13 | 11.4 | |
| 69 | 12 | 10.6 | |
| 70 | 11 | 9.7 | |
| 71 | 10 | 8.8 | |
| 72 | 9 | 7.9 | |
| 73 | 8 | 7.0 | |
| 74 | 7 | 6.2 | |
| 75 | 6 | 5.3 | |
| 75 | 5 | 4.4 | |
| 77 | 4 | 3.5 | |
| 78 | 3 | 2.6 | |
| 79 | 2 | 1.8 | |
| 80 | 1 | 0.9 | |
| 81 | 20 | 22.0 | |
| 82 | 19 | 20.9 | |
| 83 | 18 | 19.8 | |
| 84 | 17 | 18.7 | |
| 85 | 16 | 17.6 | |

(continued)

| Number of combination | Selected API dose per single dosage unit [mg] | Approximate minimum area to be covered by a single dosage unit [cm$^2$] | Target combined API dose [mg] |
|---|---|---|---|
| 86 | 15 | 16.5 | |
| 87 | 14 | 15.4 | |
| 88 | 13 | 14.3 | |
| 89 | 12 | 13.2 | |
| 90 | 11 | 12.1 | |
| 91 | 10 | 11.0 | |
| 92 | 9 | 9.9 | |
| 93 | 8 | 8.8 | 100 mg or less |
| 94 | 7 | 7.7 | |
| 95 | 6 | 6.6 | |
| 96 | 5 | 5.5 | |
| 97 | 4 | 4.4 | |
| 98 | 3 | 3.3 | |
| 99 | 2 | 2.2 | |
| 100 | 1 | 1.1 | |

Preferred combinations of dose and area according to the present invention, when the area of the dosage unit is selected first, can be derived from the subsequent Table 2. The target combined API dose refers to the approximate dose of the selected API not to be exceeded if multiple of said dosage unit are combined to cover all non-keratinized parts of a mouth. The target combined API dose can be the maximum dose of an API used by the skilled person to induce a respective therapeutic effect according to a risk-benefit balance, accordingly a toxic effect is thereby prevented.

Alternatively, the target combined API dose can be limited to remain below the minimum dose of an API used by the skilled person to induce a primary, therapeutic effect. Accordingly, achieving a secondary therapeutic effect at a target combined API dose below that required for a primary therapy effect is enabled. The secondary therapeutic effect at a API dose below the dose required for a primary therapeutic effect can occur by a different mechanism than the primary therapeutic effect.

In a preferred embodiment the area of the non-keratinized parts of the mouth of a subject is approximately 110 cm$^2$. Likewise this area is about 45 - 110 cm$^2$ for a not full-grown human subject The approximate maximal selectable dose of a dosage unit according to the invention can be calculated by dividing the approximate area of the non-keratinized parts of the mouth (A), which is in a preferred embodiment 110 cm$^2$, by the selected area of the dosage unit (a) in a first step. In a second step the target combined API dose (D) is divided by the quotient y obtained in the first step, yielding the approximate maximum selectable API dose per single dosage unit (d). In a preferred embodiment, the analgesic and/or sedative comprised is propofol and the target combined API dose is 175 mg. In a preferred embodiment, the subject is a human, the API comprised is propofol, the area of the non-keratinized parts in the mouth is about 110 cm$^2$ and the target combined API dose is 175 mg.

This calculation can be visualized as:

Step 1 (selection of a):

$$A / a = y$$

Step 2 (determination of d):

$$D / y = d$$

With

A: area of the non-keratinized parts of the mouth, in a preferred embodiment this is 110cm$^2$ for a human subject [cm$^2$]
a: selected area of the dosage unit [cm$^2$]
y: quotient of the area of the non-keratinized parts of the mouth divided by the selected area of the dosage unitD: value of the target combined API dose [mg]
d: approximate maximum selectable API dose per single dosage unit [mg]

Table 2: Preferred combinations of selected dose and area covered per single dosage unit, when the area of the dosage unit is selected first for different target combined API doses.

| Number of combination | Selected area of a single dosage unit [cm$^2$] | Approximate maximum selectable API dose per single dosage unit [mg] | Target combined API dose [mg] |
|---|---|---|---|
| 101 | 20 | 36.4 | |
| 102 | 19 | 34.5 | |
| 103 | 18 | 32.7 | |
| 104 | 17 | 30.9 | |
| 105 | 16 | 29.1 | |
| 106 | 15 | 27.3 | |
| 107 | 14 | 25.5 | |
| 108 | 13 | 23.6 | |
| 109 | 12 | 21.8 | 200 mg or less |
| 110 | 11 | 20 | |
| 111 | 10 | 18.3 | |
| 112 | 9 | 16.4 | |
| 113 | 8 | 14.5 | |
| 114 | 7 | 12.7 | |
| 115 | 6 | 10.9 | |
| 116 | 5 | 9.1 | |
| 117 | 4 | 7.3 | |
| 118 | 3 | 5.5 | |
| 119 | 2 | 3.6 | |
| 120 | 1 | 1.8 | |

(continued)

| Number of combination | Selected area of a single dosage unit [cm$^2$] | Approximate maximum selectable API dose per single dosage unit [mg] | Target combined API dose [mg] |
|---|---|---|---|
| 121 | 20 | 31.8 | 175 mg or less |
| 122 | 19 | 30.2 | |
| 123 | 18 | 28.6 | |
| 124 | 17 | 27.0 | |
| 125 | 16 | 25.5 | |
| 126 | 15 | 23.9 | |
| 127 | 14 | 22.3 | |
| 128 | 13 | 20.7 | |
| 129 | 12 | 19.1 | |
| 130 | 11 | 17.5 | |
| 131 | 10 | 15.9 | |
| 132 | 9 | 14.3 | |
| 133 | 8 | 12.7 | |
| 134 | 7 | 11.1 | |
| 135 | 6 | 9.5 | |
| 136 | 5 | 8.0 | |
| 137 | 4 | 6.4 | |
| 138 | 3 | 4.8 | |
| 139 | 2 | 3.2 | |
| 140 | 1 | 1.6 | |
| 141 | 20 | 27.3 | 150 mg or less |
| 142 | 19 | 25.9 | |
| 143 | 18 | 24.5 | |
| 144 | 17 | 23.2 | |
| 145 | 16 | 21.8 | |
| 146 | 15 | 20.5 | |
| 147 | 14 | 19.1 | |
| 148 | 13 | 17.7 | |
| 149 | 12 | 16.4 | |
| 150 | 11 | 15.0 | |
| 151 | 10 | 13.6 | |
| 152 | 9 | 12.3 | |
| 153 | 8 | 11 | |
| 154 | 7 | 9.5 | |
| 155 | 6 | 8.2 | |
| 156 | 5 | 6.8 | |

(continued)

| Number of combination | Selected area of a single dosage unit [cm$^2$] | Approximate maximum selectable API dose per single dosage unit [mg] | Target combined API dose [mg] |
|---|---|---|---|
| 157 | 4 | 5.5 | |
| 158 | 3 | 4.1 | |
| 159 | 2 | 2.7 | |
| 160 | 1 | 1.4 | |
| 161 | 20 | 22.7 | |
| 162 | 19 | 21.6 | |
| 163 | 18 | 20.5 | |
| 164 | 17 | 19.3 | |
| 165 | 16 | 18.2 | |
| 166 | 15 | 17.0 | |
| 167 | 14 | 15.9 | |
| 168 | 13 | 14.8 | |
| 169 | 12 | 13.6 | |
| 170 | 11 | 12.5 | 125 mg or less |
| 171 | 10 | 11.4 | |
| 172 | 9 | 10.2 | |
| 173 | 8 | 9.1 | |
| 174 | 7 | 8.0 | |
| 175 | 6 | 6.8 | |
| 175 | 5 | 5.7 | |
| 177 | 4 | 4.5 | |
| 178 | 3 | 3.4 | |
| 179 | 2 | 2.3 | |
| 180 | 1 | 1.1 | |

(continued)

| Number of combination | Selected area of a single dosage unit [cm$^2$] | Approximate maximum selectable API dose per single dosage unit [mg] | Target combined API dose [mg] |
|---|---|---|---|
| 181 | 20 | 18.2 | 100 mg or less |
| 182 | 19 | 17.3 | |
| 183 | 18 | 16.4 | |
| 184 | 17 | 15.5 | |
| 185 | 16 | 14.5 | |
| 186 | 15 | 13.6 | |
| 187 | 14 | 12.7 | |
| 188 | 13 | 11.8 | |
| 189 | 12 | 10.9 | |
| 190 | 11 | 10.0 | |
| 191 | 10 | 9.1 | |
| 192 | 9 | 8.2 | |
| 193 | 8 | 7.3 | |
| 194 | 7 | 6.4 | |
| 195 | 6 | 5.5 | |
| 196 | 5 | 4.5 | |
| 197 | 4 | 3.6 | |
| 198 | 3 | 2.7 | |
| 199 | 2 | 1.8 | |
| 200 | 1 | 0.9 | |

In an embodiment, the dosage unit has an area selected from the ranges of at least 1 cm$^2$, at least 2 cm at least 3 cm$^2$, at least 4 cm$^2$, at least 5 cm$^2$, at least 6 cm$^2$, at least 7cm$^2$, at least 8 cm$^2$, at least 9 cm$^2$, at least 10 cm$^2$, at least 11 cm$^2$ or at least 12 cm$^2$, preferably at least 6 cm$^2$.

The term "film" in the context of the present invention is a solid or semisolid carrier suited to bind, absorb, or otherwise receive an API. A "film" in the context of the present invention can be used to deliver an API, particularly to deliver an API upon adhesion, such as mucoadhesion. The film is preferably a layer, in particular a cohesive, solid or gelatinous layer. The film can be formed by casting or otherwise applying a composition containing a polymer which is suitable for forming a film solved or dispersed in a solvent and, optionally, further ingredients such as a plasticizer and the API onto a surface. The film can also be produced by extrusion, semi-solid casting method, rolling or printing, such as 3D-printing. Preferably, dosage units according to the invention can be obtained from a film.

The term "mucoadhesive" in the context of the present invention refers to the ability of a dosage unit to be affixed to a mucosa, in particular the mucosa of the oral cavity, more particularly the non-keratinized parts of the oral mucosa. Mucoadhesiveness of a dosage unit enables close adherence of the dosage unit to the mucosa of the oral cavity. Advantageously, a facilitated adherence of the dosage unit to the mucosa of the oral cavity can increase and improve the proportion of an API comprised in the dosage unit that is resorbed over the mucsoa by improving. Facilitated adherence to the mucosa can improve the penetration and/or permeation of the API comprised in the dosage unit. In a preferred embodiment, delivery of the comprised API occurs preferentially and/or almost exclusively via non-keratinized parts of the mouth, more preferentially the delivery of the compound of the comprised API occurs exclusively via non-keratinized parts of the mouth. In a preferred embodiment, the time of mucoadhesiveness of the dosage unit can be selected. Selection of the time of mucoadhesion can be used to limit the residence time of the dosage unit.

The term "pain" in the context of the present invention can refer to a distressing feeling arising from a sensory or emotional experience. Pain can also refer to a complex sensory sensation triggered by nociceptors of the peripheral nervous system. The perception of pain is processed and interpreted in the central nervous system (CNS). There are close interactions between the perception of pain and the mental state of the subject. Pain can be caused by an intense or harmful stimulus. Pain can refer to a symptom of a disease such as migraine and/or be a disease itself. Pain may be perceived over an extended period of time, whereby the underlying cause cannot or hardly be treated, or cannot be identified at all.

An example of pain as disease is chronic pain or a pain syndrome. In such cases, the body of the subject may have learned of states of pain. Repeated states of pain can lead to more intense and longer pain sensation, as the pain threshold is lowered. Therefore, an early and sufficient treatment of pain is generally recommended. However, in many countries including Germany the treatment of pain is often inadequate. This may be because of a strong cautious state of mind not to develop an addiction or apply an overdose of pain medication.

Pain can be separated depending on the triggering sensation into physiological pain, neuropathic pain or pain as consequence of functional problems in the body of a subject. In physiological pain, pain can be typically perceived by nociceptors. In neuropathic pain, pain can occur as a result of damage to the nervous system, such as after a virus infection or amputation. Pain as consequence of functional problems in the body of a subject can be perceived as a result of some parts of the body being functionally defective or the reaction of the body to external stimuli is defective. For example, a defective regulation of blood circulation may lead to migraine.

In physiological pain, the perception of pain occurs in so-called pain receptors or nociceptors. Nociceptors can be free nerve endings. Nociceptors can react to different stimuli which can result in the perception of pain. Stimuli that nociceptors react to qualitatively, include thermal stimuli such as heat or cold, mechanical stimuli such as pressure or damage or chemical stimuli such as inflammation, acids, toxins. There is a threshold level for the stimuli in order to induce the perception of pain in the nociceptors. Furthermore, nociceptors do not adapt, such as a stimulus that is repeated over and over again, does not induce a reduction of excitability. Therefore stimuli that can qualitatively induce the perception of pain, may not be strong enough to induce the perception of pain. Accordingly, pain can also be the perception of pain. The excitability of nociceptors may be modulated by molecules including so-called pain mediators. Typically, the excitability of nociceptors is increased. Pain mediators include prostaglandins, bradykinins and serotonin. The excitability of nociceptors can also be increased in cases of oxygen hypoxia in the tissue, a reduction of the pH value, such as in cases of a $CO_2$ increase in the tissue, or a change of the concentration of electrolytes in the blood.

Pain can also be classified according to its duration into acute and chronic pain. Acute pain can have a signaling effect and indicate a problematic cause enabling removal of the cause the treatment of the pain. Treatment of acute pain can be performed for example by using API according to the WHO pain relief ladder or by applying a local or systemic sedative.

Depending on the intensity of the acute pain treatment of pain can include API of different strength or combination of different API. Pain that is perceived for more than 6 months can be referred to as chronic pain. Furthermore, pain that occurs repeatedly, such as a migraine pain, on at least 15 days per month can be considered chronic pain. In chronic pain, the pain generally exists as a separate disease independent of the originally disease. In chronic pain, the perception of pain has lost its signaling effect. Chronic pain can for example be treated by applying API according to the WHO pain relief ladder or by applying a local or systemic sedative. Insufficient of absent treatment of chronic pain can lead to the development of a pain memory. Pain as a disease can result in secondary effects such as sleep disorders, reduced performance at work, incapacity to work or significant limitation of physical and mental resilience and onset of depression. In such cases of secondary effects, treatment by medication alone can be insufficient to restore the quality of life of a subject infected. Advantageously dosage units according to the present invention enable a better early treatment of pain, since they prevent overdosing. Therefore the generation of chronic pain can be prevented.

Pain can also be classified according to its quality and intensiveness. Pain can be classified according an affective pain quality or a sensory pain quality. Affective pain quality refers to the subjective perception of the pain, such as violent, paralyzing, agonizing, martyring or terrible. Sensory pain perception refers to the actual perception of the pain for example as pulling, dull, drilling, stabbing, stinging, pressing, burning or knocking.

The perception of pain in a subject can also be dysfunctional. A subject with dysfunctional pain perception can be more sensitive to the perception of pain or the subject can be less sensitive to the perception of pain. Dysfunctional perception of pain can for example occur in a subject inflicted with hyperalgesia, hypalgesia or allodynia. Subjects inflicted with a condition that increases their sensitivity to pain can be more prone to overdosing pain medication.

Pain can also be classified according to the location where it is perceived. Examples include headache, such as migraine, cluster headache, tension headache or paroxysmal hemicranias, facial pain, such as trigeminal neuralgia, or Costen syndrome, toothache, sore throat, such as pharyngitis or angina tonsillaris, neck pain such as cervical spine syndrome, cervical brachialgia or cervicocephalgia, torso pain, such as thoracic pain, such as retrosternal pain in angina pectoris or myocarcial infarction, back pain, such as lumboischialgia, sciatica, thoracic spine syndrome, lumbar spine syndrome, piriformis syndrome, sacralgia or coccygodynia, flank pain, groin pain, abdominal pain, such as a pain in the upper abdomen or a pain in the lower abdomen, pain in the extremities, such as leg pain, arm pain or shoulder pain. In other cases pain can be perceived at a location different from that of the causative stimulus. Pain can be perceived at a different location from the site of the causative stimulus in so-called referred pain. In referred pain the pain may be projected onto a specific dermatome or myotome of the corresponding segment of the spinal cord. Examples include periumbilical pain in early stages of appendicitis or right shoulder pain in subjects inflicted with cholecystitis.

Moreover, pain can be classified into mild, moderate and strong pain. Pain can be classified according to standardized intensity scales known to the skilled person. Pain scales include numerical scales such as the numerical rating scale (NRS-11), a faces pain scale such as the Wong-Baker FACES® pain rating scale, global pain scale, visual pain scale, McGill pain scale, Mankoski pain scale, color pain scale, visual analogy scale (VAS), verbal numerical rating scale (VNRS), verbal description scale (VDS) or pediatric pain scale. For example in the NRS-11, a rating of 0 refers to no pain, a rating of 1 - 3 refers to mild pain, a rating of 4 - 6 refers to moderate pain and a rating of 7 - 10 in this scale refers to strong or severe pain.

Headaches can be broadly classified in "primary", "secondary" and "painful cranial neuropathies, other facial pain and other headaches" (Cephalalgia. 2018 Jan;38(1):1-211). Examples of primary headaches include migraine, tension headache or cluster headache. Examples of secondary headaches include headaches from infection, inflammation, head injury, trauma, stroke, vascular disorders, brain bleeding and tumors. Examples of cranial neuropathies, facial pain and other headaches include pain attributed to a lesion or disease of the trigeminal nerve and pain attributed to a lesion or disease of the glossopharyngeal nerve.

Pain from migraine can be perceived as mild, moderate or severe. Migraine pain can be episodic. Migraine pain can become chronic, leading to a so-called chronic migraine. Chronic migraine can also be diagnosed upon occurrence of at least 8 days with migraine per month. Migraine headaches can be further classified into migraines without aura or into migraines with aura. Migraine without aura can be characterized by moderate to severe throbbing pain, whereby the pain lasts from 4 - 72 h. A migraine without aura occurs suddenly and generally unilateral and worsens by movement of the inflicted subject. Subjects inflicted with migraine without aura can also experience nausea, loss of appetite, photophobia or phonophobia. In migraine with aura, the inflicted subject experiences an aura phase that can last up to 60 minutes and can include visual or sensory disturbances which proceeds into migraine headaches. Male and females can be inflicted with migraine, whereby females have been found to be more affected than males. A particular form of female migraine is menstrual migraine. Also children can be inflicted with migraine, in particular pediatric migraine. A migraine can also be classified as refractory or intractable. Refractory and intractable migraine include migraine that does not respond to conventional migraine treatment, has a high frequency, a severe disability or a combination of these features. Refractory or intractable migraine can be diagnosed according to guidelines such as guidelines from the European Headache Federation or the Refractory Headache Special Interest Section (RHSIS) of the American Headache Society (AHS). Acute confusional migraine (ACM) is a rare form of migraine disorder, in which the inflicted subject presents with a confusional state. ACM can also occur in children. The severity of a migraine attack can be scored using pain scales such as VAS or the NRS_11. In one embodiment, migraine can be a neurological disorder or a nervous system disease. In this embodiment, the inflicted subject can experience symptoms such as aura, nausea, photophobia, hemiparesis, phonophobia, but does not experience pain, in particular does not experience headache. This form of migraine can also be referred to as migraine with aura with no headache. It can also be distinguished from a migraine with a purely visual aura without headache, also referred to as ocular migraine. The treatment of migraine as neurological disorder can be the same as for migraine as a pain disease. Migraine attacks can be treated with API or medication selected from the group of beta-blockers, anti-convulsants, tricyclics, calcium channel blockers, triptans, ditans such as lasmiditan, dihydroergotamine (DHE), NSAIDs, dopamine antagonists, ergot alkaloids, opioids, glucocorticoid steroids, anti-depressants, anti-seizure drugs such as topiramate or sodium valproate, onabotulinum toxin A, acetaminophen or others. The need to treat every migraine attack in combination with a reduced efficacy of backup medication, can determinate the occurrence of medication overdose.

The term "treatment of pain" as used in the context of this invention can refer to the protection from pain, the protection from pain perception, the prevention of pain, the prevention of pain perception, the reduction of pain, the reduction of pain perception and an increase in the welfare of the treated subject. The terms protection from pain and protection from pain perception includes treatment that is initiated before the onset of pain or onset of pain perception and that is continued after onset of pain or onset of pain perception. The terms prevention from pain and prevention from pain perception includes treatment that is initiated before the onset of pain or onset of pain perception. The term "treatment" also includes an alleviation of the symptoms, a delay of onset of symptoms, reduction of symptoms and a cure of pain. The term reduction of pain or reduction of pain perception includes treatment that is initiated with or after onset of pain or onset of pain perception. The term "treatment of pain" as used in the context of this invention can also refer to an improvement of the physical and/or mental state of the subject, i.e. the welfare of the treated subject. An improvement in the physical state includes improved reaction, strength and endurance or ability to work. An improvement in the mental state includes improved resilience, concentration and emotional stability. In an embodiment, pain, in particular migraine pain, is treated if the subject is pain-free 2 h after the treatment. Pain can for instance be treated by applying at least one anesthetic or at least one analgesic. Pain can also be treated by applying at least one anesthetic and at least one analgesic. Applying an anesthetic in combination with an analgesic can be referred to as a procedure called analgosedation.

The term "metabolic disorders" or "metabolic disease" as used in the context of this invention can refer to any disease or disorder that is caused by an alteration in at least one metabolic process in the body of a subject. A metabolic disorder can refer to a congenital or inherited metabolic disease and can describe a physiological consequence resulting from a gene anomaly, in particular a single gene anomaly, even more particular a single gene anomaly in an autosomal recessive gene. An example of a congenital metabolic disease is type 1 diabetes mellitus. A metabolic disorder can also refer to an acquired metabolic disease and can describe a physiological consequence resulting from a different primary disease. An example of an acquired metabolic disease is a metabolic disease resulting from a renal disease.

A metabolic disorder can be classified according to the metabolite or substrate of a metabolic process in the body the processing of which is affected as a result of the metabolic disease. A metabolic disorder can be classified according to the metabolic process affected as a result of the metabolic disease.

In one embodiment of the invention a metabolic disorder can refer to a disorder selected from the group of acid-base imbalances, metabolic brain diseases, disorders of calcium metabolism, DNA repair-deficiency disorder, glucose metabolism disorders, hyperlactatemia, iron metabolism disorders, lipid metabolism disorders, malabsorption syndromes, metabolic syndrome X, inborn error of metabolism, mitochondrial diseases, phosphorus metabolism disorders, porphyrias, proteostasis deficiencies, metabolic skin diseases, wasting syndrome and water-electrolyte imbalances.

In another embodiment a metabolic disorder can be selected from the group of adenosine monophosphate deaminase deficiency type 1, adrenogenital syndrome, 3-methylglutaconic aciduria, alpha-1 antitrypsin deficiency, alkaptonuria, diabetes mellitus, neutral lipid storage disease, cystinuria, Erythropoietic protoporphyria, food intolerances, galactosemia, gout, glutaric aciduria type 1, glycogen storage disease, urea cycle disorder, Hashimoto's thyroiditis, Hartnup disease, acrodermatitis enteropathica, hereditary hemochromatosis, homocystinuria, hyperlipidemia, hypophosphatasia, hypothyroidism, ketoacidosis, ketosis, lecithin cholesterol acyltransferase deficiency, acetonuria, Lesch-Nyhan syndrome, sphingolipidoses, Maroteaux-Lamy syndrome, methylmalonaziduria, Addison's disease, primary aldosteronism, Cushing's disease, Fabry's disease, Gaucher's disease, Hunter syndrome, Morquio syndrome, Wilson's disease, cystic fibrosis, osteoporosis, phenylketonuria, porphyries and thesaurismosis.

The term "treatment of a metabolic disorder" as used in the context of this invention can refer to the protection from a metabolic disorder, the prevention of a metabolic disorder, the reduction of the symptoms of a metabolic disorder, and an increase in the welfare of the treated subject suffering from a metabolic disorder. The term protection from a metabolic disorder includes treatment that is initiated before the onset of a metabolic disorder and that is continued after onset of a metabolic disorder. The terms prevention from a metabolic disorder includes treatment that is initiated before the onset of a metabolic disorder. The term "treatment" also includes an alleviation of the symptoms, a delay of onset of symptoms, reduction of symptoms and a cure of a metabolic disorder. The term reduction of a metabolic disorder includes treatment that is initiated with or after onset of a metabolic disorder. The term "treatment of a metabolic disorder" as used in the context of this invention can also refer to an improvement of the physical and/or mental state of the subject, i.e. the welfare of the treated subject. An improvement in the physical state includes improved reaction, strength and endurance or ability to work. An improvement in the mental state includes improved resilience, concentration and emotional stability. A metabolic disorder can for instance be treated by applying at least one enzyme absent or dysfunctional in the metabolic disorder. A metabolic disorder can also be treated by applying an agent that reduces the amount of the specific substrate of an enzyme absent or dysfunctional in the metabolic disorder. A metabolic disorder can also be treated by applying at least one enzyme absent or dysfunctional in the metabolic disorder and applying an agent that reduces the amount of the specific substrate of an enzyme absent or dysfunctional in the metabolic disorder.

The term "central nervous system (CNS) disease" "nervous system disease" or "neurological disorder" can refer to a disease or disorder that affects the structure of function of the brain and/or spinal cord. CNS diseases can be classified according to the cause of the disease. CNS diseases can be accordingly separated into autoimmune CNS diseases, trauma-related CNS diseases, infection-related CNS diseases, degenerative CNS diseases, structural defect-related CNS diseases, cancer-related CNS diseases, inherited CNS diseases, metabolism-related CNS diseases, neuropathic CNS diseases and stroke-related CNS diseases.

An autoimmune CNS disease can be selected from the group of acute demyelinating encephalomyelitis, multiple sclerosis, neuritis nervi optici, neuromyelitis optica, radiologically isolated syndrome, and clinically isolated syndrome. A degenerative CNS disease can be selected from the group of Alzheimer's disease, amyotrophic lateral sclerosis, Friedreich's ataxia, Huntington's disease, Lewy body disease, Parkinson's disease and spinal muscular atrophy.

In an embodiment the CNS disease the CNS diseases is a neuropathic CNS disease. In this embodiment nerve structures in the central nervous system or the central nervous system and the peripheral nervous system are damaged. A neuropathic CNS disease can be caused by a lesion in a primary process of the CNS, such as a lesion in the spinal cord, brain stem, thalamus, in subcortical structures and in the cortex. In this embodiment the neuropathic CNS disease can be accompanied by neuropathic pain or the perception of neuropathic pain. In this embodiment the subject inflicted with a neuropathic CNS disease can also experience non-neuropathic pain, in particular physiological pain and/or pain as consequence of functional problems in the body of the subject. In this embodiment, the CNS disease can be a migraine with aura during which the subject does not experience pain. In this embodiment, the migraine is a neurological disorder. In this embodiment, the migraine can also be referred to as silent or acephalgic migraine. In acephalgic migraine the subject inflicted can experience an aura, nausea, photophobia, hemiparesis, phonophobia or other migraine symptoms, but does not experience pain, in particular does not experience headache. The migraine can also be referred to as ocular migraine or retinal migraine. In ocular migraine the subject inflicted can experience a visual aura or changes in vision without experiencing headache. In retinal migraine the subject inflicted can experience repeated bouts of shortlasting, diminished vision or blindness in one eye, not both eyes, without experiencing headache.

In another embodiment a CNS disease can be selected from the group of addiction, arachnoid cysts, attention deficit/hyperactivity disorder (ADHD), autism, bipolar disorder, catalepsy, depression, encephalitis, epilepsy/seizures, infection, locked-in-syndrome, meningitis, migraine, multiple sclerosis, myelopathy, neurophatic pain Tourette's and neurodegenerative disorders including Alzheimer's, Huntington's disease and Parkinson's.

The term "anesthetic" as used in the context of this invention can refer to an API that results in a temporary loss of sensation and/or awareness and that has anxiolytic, hypnotic, sedative and/or narcotic effect. The term anesthetic can be used to include anxyolitcs, hypnotics, sedatives, and/or narcotics. Likewise, an "anesthetic dose" or "therapeutic anesthetic dose" can refer to the dose of an API that exerts an anxiolytic, hypnotic, sedative and/or narcotic effect. An example of an anesthetic is propofol.

The term "subject" as used in the context of this invention can refer to humans and non-human animals. Preferably "subject" refers to a human. The term "subject" can also refer to a full-grown human, a full-grown non-human animal, a not full-grown human and/or a not full-grown non-human animal. A full-grown human can be an adult human. A non-full grown human can be a child. Preferably the term "subject" refers to a full-grown human. Further preferably, the term "subject" refers to a not full-grown human. Most preferably, the term refers to full-grown human and/or a not full-grown human.

The term "mouth" as used in the context of this invention refers to the mouth, in particular the tissue of the mouth, of a subject. The term "mouth" can also refer to the body cavity, the oral cavity, and the surrounding soft tissue in the head of a subject that forms the uppermost part of the digestive tract. In a preferred embodiment, in which the subject is a full-grown human, the mouth is the orifice through which food and air enter the mouth. In this preferred embodiment, the parts of the mouth include the oral fissure, which is surrounded by the lips, the vestibule of the mouth, which is located between the lips and the cheeks on the one hand, and between the upper and lower jaw and rows of teeth on the other hand and the oral cavity, with the base or floor of the mouth, the tongue and the hard and soft palate.

The term "mucosa" in the context of this invention refers to mucous membranes that line various cavities in the body of an animal and cover the surface of internal organs. In the context of the oral cavity, the term refers to the oral mucous membrane that almost continuously lines the oral cavity and provides the outermost moist lining of the oral cavity. The oral mucosa can be perforated by ducts or salivary glands. It fulfills essentially 3 different functions: secretion, sensation and protection. The oral mucosa is continuous with the skin, but is different in composition and structure, due to the varied functions in the oral cavity. The oral mucosa is composed of stratified squamous epithelium overlying a connective tissue proper or lamina propria with possibly a deeper submucosa. A basement membrane can be located between the squamous epithelium and the lamina propria. Typically, the epithelium constitutes the outer layer of body surfaces, the linings of alimentary canal and the walls of hollow structures including body cavities. The epithelium in the oral mucosa can also be referred to as keratinocytes because the cells in the epithelium can produce keratin either in a physiological state at physiological levels or at higher levels when the epithelium becomes traumatized, even if the keratinocytes did not previously produce keratin. A physiological level also includes absence of keratin production by keratinocytes.

Moreover, three different types of mucosa can be separated in the oral cavity based on histological and functional features lining mucosa, masticatory mucosa and specialized mucosa.

The lining mucosa type can be found in the lips, the buccal mucosa, the labial mucosa, the alveolar mucosa, the ventral tongue surface, the floor of the mouth, the oropharynx and the soft palate. Its' clinical appearance can be characterized by softer surface texture, most surface, and the ability to stretch and be compressed, thereby acting as a cushion. Its' microscopic appearance can be characterized by the presence of non-keratinized epithelium with smooth interface, and few rete ridges and connective tissue papillae, but by the presence of elastic fibers in the lamina propria and submucosa. Rete ridges may also be referred to as rete processes or rete pegs and describe epithelial extensions that project into the underlying connective tissue in the mucosa.

The masticatory mucosa type can be found in the gingiva, the hard palate and the dorsal tongue surface. Its' clinical appearance can be characterized by a rubbery surface texture and resilience, wherefore it can serve as a firm base. Its' microscopic appearance can be characterized by the keratinized epithelium and the interdigitated interface with many rete ridges and connective tissue papillae with a thin layer of submucosa or none at all. When the masticatory mucosa overlies bone, with or without submucosa, the masticatory mucosa can increase the firmness of the tissue.

The specialized mucosa type can be found in the dorsal tongue surface, in particular the circumvallate papillae, the folate papillae, the fungiform papillae and the filiform papillae. Its' clinical appearance can be characterized by their association with lingual papillae. Its' microscopic appearance can be characterized as similar to masticatory mucosa. Specialized mucosa can be keratinized. Moreover, it can be characterized by its' presence at discrete structures of epithelium and lamina propria, such as papillae or taste buds.

The ratio of lining mucosa, masticatory mucosa and specialized mucosa can vary in between subjects of an animal species, e.g. the ratio can vary because of different developmental or growing phases between such subjects. The ratio can also vary between subjects of different species. In a preferred embodiment, in which the subject is a full-grown human subject, the lining mucosa covers about 60%, the masticatory mucosa covers about 25% and the specialized mucosa covers about 15% of the parts of the mouth and/or oral cavity.

Moreover, based on the presence of keratin in the epithelium, the parts of the mouth and/or the oral cavity can be grouped into, keratinized oral mucosa and non-keratinized oral mucosa. Keratins are a diverse group of structural proteins that among other functions form the intermediate filament network and which can provide further structural integrity.

Non-keratinized mucosa refers to mucosa in which the epithelium is not-keratinized. A non-keratinized mucosa epithelium can refer to an epithelium in which the surface cells and/or the outermost cell layer are living cells. A non-keratinized epithelium can also refer to the absence of keratin in the epithelial cells. A non-keratinized epithelium can also refer to an epithelium that is pervious to water. A non-keratinized epithelium can also refer an epithelium that provides moderate protection against abrasions. A non-keratinized epithelium can also refer to the lining of the buccal cavity, the pharynx and/or the oesophagus.

The term "keratinized oral mucosa" refers to mucosa in which the epithelium is keratinized. A keratinized epithelium can include ortho-keratinized and/or para-keratinized mucosa. A keratinized epithelium can refer to the epidermis of a land vertebrate. A keratinized epithelium can refer to an epithelium in which the surface cells and/or outermost cell layer are dead cells. A keratinized epithelium can refer to an epithelium in which the surface cells and/or outermost cell layer are cells in which the cytoplasm is largely replaced by keratin. A keratinized epithelium can refer to an epithelium which is impervious to water. A keratinized epithelium can refer to an epithelium which provides good protection against abrasions.

A keratinized oral mucosa can include para-keratinized mucosa and/or ortho-keratinized mucosa. Masticatory mucosa can include para-keratinized and/or orthokeratinized mucosa. Para-keratinized mucosa can refer to partial keratinization or a keratinized mucosa in which the cells forming the keratinized epithelium comprise keratin and nuclei. Ortho-keratinized mucosa can refer to full keratinization or a keratinized mucosa in which the cells forming the keratinized epithelium comprise keratin and are devoid of nuclei.

The ratio of the different types of mucosa with regard to how much of the parts of the mouth they cover varies. The ratio of non-keratinized mucosa and keratinized mucosa can vary. The ratio can vary in between subjects of an animal species, e.g. the ratio can vary because of different developmental or growing phases between such subjects. The ratio can also vary between subjects of different species. In a preferred embodiment, in which the subject is a full-grown human subject, the ratio of non-keratinized mucosa in the parts of the mouth and/or oral cavity are in the range of 50 % - 60 % of the total surface area. In another preferred embodiment, in which the subject is a not full-grown human subject, the ratio of non-keratinized mucosa in the parts of the mouth and/or oral cavity are in the range of 50 - 70 % of the total surface area of the parts of the mouth.

In an embodiment the parts of the mouth have an area of about 220 cm$^2$. In this embodiment, the non-keratinized parts cover an area of about 150 cm$^2$, about 130 cm$^2$, or about 110 cm$^2$.

In a preferred embodiment, the mouth is the mouth of a full-grown human. In this embodiment the parts of the mouth can have an area of about 220 cm$^2$. In this embodiment, the non-keratinized parts can cover an area of about 150 cm$^2$, about 130 cm$^2$, or about 110 cm$^2$.

In a different preferred embodiment, the mouth is the mouth of a not full-grown human. In this embodiment the parts of the mouth can have an area of up to 220 cm$^2$. In newborn not full-grown human subjects the parts of the mouth can have an area of about 90 cm$^2$ or more. Depending on the developmental stage of the not full-grown human subject, the parts of the mouth can have an area selected in the rage from 90 - 220 cm$^2$. In this embodiment the parts of the mouth can have an area that is a function of the growth status of the not full-grown human subject. In particular, the area of the mouth is equal to or smaller than that of a full-grown human. In this embodiment, the non-keratinized parts can cover a surface area of 30 to 90 % of the the total parts of the mouth, particularly 40 to 80 % of the total parts of the mouth, more particular 50 to 70 % of the total parts of the mouth, most particularly around 60 % of the total parts of the mouth. Depending on the developmental stage of the not full-grown human the non-keratinized parts of the mouth can cover an area of 45 - 150 cm$^2$, of 45 - 130 cm$^2$ or of 45 -110 cm$^2$, preferably of 45 - 110 cm$^2$.

In a further preferred embodiment, the mouth is the mouth of a full-grown non-human animal, in particular an agricultural animal. Agricultural animals can be animals that are or were used in an agricultural or economical context including animals bred for consumption, farmed ruminants or livestock farming. Agricultural animals can be camels, llamas, horses, donkeys, mules, cattle, buffalo, oxen, pig, sheep, goats and dogs. In this embodiment, the part of the mouth can have an area of more than 220 cm$^2$, in particular an area of 220 cm$^2$ to 500 cm$^2$. In this embodiment the the ratio of non-keratinized mucosa in the parts of the mouth and/or oral cavity are in the range of 20 - 80 %, more particular in the range of 30 - 70 %.

The term "to exceed an analgesic or sedative therapeutic dose" can refer to a dose of a respective analgesic or sedative that is administered via at least one dosage unit which is higher than the highest therapeutic dose of said analgesic or sedative. The term can also refer to an overdose of said analgesic or sedative. Therapeutic doses of analgesics and sedatives are known to the skilled person. The term can also refer to a lethal dose of an analgesic and/or sedative. The term can also refer to a toxic dose, in particular a lethal toxic dose, of an analgesic or sedative if no therapeutic dose of the respective analgesic or sedative is known to the skilled person. The term "to exceed an analgesic or sedative therapeutic dose" can also refer to a dose that achies a systemic dose higher than the therapeutic analgesic and/or sedative serum level of the respective API. The serum level of an analgesic or a sedative can be determined for example by therapeutic drug monitoring from blood samples of the subject and are known to the skilled person. The term "to exceed an analgesic or sedative therapeutic dose" can also refer to a dose of a respective analgesic and/or sedative that is below the administered analgesic and/or sedative therapeutic dose but achieves a systemic dose higher than the therapeutic analgesic and/or sedative serum level of the respective API. The term "to exceed an analgesic or sedative therapeutic dose" can also refer to a dose of a respective analgesic and/or sedative that achieves a toxic systemic dose, in particular a lethal toxic dose, of the respective API.

The term "to exceed a therapeutic dose" as used in the context of this invention can refer to a dose of an API that is administered via at least one dosage unit which is higher than the highest therapeutic dose of said API. The term can also refer to an overdose of an API. Therapeutic doses of API are known to the skilled person. If a maximum therapeutic dose of an API is exceeded a toxic effect can occur. The term "to exceed a therapeutic dose" can also refer to a lethal dose of an API. The term can also refer to a toxic dose, in particular a lethal toxic dose, of an API if no therapeutic dose of the respective API is known to the skilled person. The term "to exceed a therapeutic dose" can also refer to a dose that achieves a systemic dose higher than the therapeutic systemic serum or plasma level of the respective API. The systemic dose of an API can be the serum level of the API. The serum level of an API can be determined for example by therapeutic drug monitoring from blood samples of the subject. Further methods for determining the systemic level of an API include blood sampling and methods such as fluorometric methods, SDS-PAGE, CE-SDS, ELISA or mass spectrometric analyses which are known to the skilled person. The term "to exceed a therapeutic dose can also refer to an administered dose of a respective API that is below the therapeutic dose, but achieves a systemic dose higher than the therapeutic serum level of the respective API. The term "to exceed a therapeutic dose" can also refer to a dose of a respective API that achieves a toxic systemic dose, in particular a lethal toxic dose, of the respective API. The term "to exceed a therapeutic dose" and can also refer to a dose of a respective API that achieves a dose that is toxic, in particular lethally toxic, and/or higher than a therapeutic serum level of the respective API over the time the API is delivered. The term "to exceed a therapeutic dose" can also refer to a dose of a respective API that is higher than a therapeutic dose over time of the respective API. An example of an analgesic and/or sedative therapeutic dose over time is the therapeutic dose of 4mg/kg (bodyweight) per hour for propofol. A further example of a hormone therapeutic dose over time is 1 IU/kg body weight per day for insulin.

The terms "a toxic effect" as used in the context of this invention can refer to the effect of a dose of an API that is administered via at least one dosage unit which is higher than the highest therapeutic dose of said API. The term can also refer to the effect of an overdose of an API. Therapeutic doses of API are known to the skilled person. A toxic effect can occur if a maximum therapeutic dose of an API is exceeded. The term "a toxic effect" can also refer to the effect of a lethal dose of an API. The term can also refer to a toxic dose, in particular a lethally toxic dose, of an API if no therapeutic dose of the respective API is known to the skilled person. The term "a toxic effect" can also refer to the effect of a dose that achieves a systemic dose higher than the therapeutic systemic serum level of the respective API. The systemic dose of an API can be the serum level of the API. The serum level of an API can be determined for example by therapeutic drug monitoring from blood samples of the subject. Further methods for determining the systemic level of an API include blood sampling and methods such as ELISA or mass spectrometric analyses which are known to the skilled person. The term "a toxic effect" can also refer to the effect of an administered dose of an API that is below the maximum therapeutic dose but achieves a systemic dose higher than the maximum therapeutic serum level of the respective API. The term "a toxic effect" can also refer to the effect of a dose of an API that achieves a toxic systemic dose, in particular a lethal toxic dose, of the respective API. The term "a toxic effect" can also refer to the effect of a dose of an API that achieves a dose that is toxic, in particular lethally toxic, and/or a therapeutic serum level of the respective API over the time the API is delivered. The term "a toxic effect" can also refer to the effect of a dose of an API that is higher than a therapeutic dose over time of the respective API. An example of a primarily harmful effect of an analgesic and/or sedative therapeutic dose over time can occur if the therapeutic dose of 4mg/kg (bodyweight) per hour for propofol is exceed. A further example of a primarily harmful effect of a hormone therapeutic dose over time can occur if the therapeutic dose of 1 IU/kg body weight per day insulin is exceeded.

Advantageously, a dosage unit according to the invention enables an outpatient use of an API liable to abuse and misuse such as overdosing without oversight by a skilled practitioner, such as a physician, a pharmacist or a nurse.

In particular in the treatment of migraine an outpatient use independent of oversight by a skilled practitioner supports a therapy with as little as possible disturbance for the patient and reduced the costs and efforts that must be invested by hospitals, pharmacies, medical practices and other institutions of the health care system.

The term "abuse" as used in the context of this invention refers to the intentional administration of an API or active substance for getting under the influence of the API or active substance for pleasure. The abusive application of an API or active substance can be done to produce euphoria. A harmful effect of API or active substance abuse can be the development of addictions and physical or psychological damages including death. Abuse or use with a harmful effect can be the intentional, permanent or sporadic overuse of an API or active substance with physical or psychological damage as a result thereof [c.f. RL 2011/83/EG or 16th amendment of the Medicinal Products Act]. Abuse can also be misuse, for example when a prescribed or by a skilled person recommended API is used in a dose higher than the dose prescribed or recommended with the intention to get under the influence of the API for pleasure. Abuse can also be overdosing, for example when an active substance is applied for pleasure in a lethal dose.

The term "misuse" as used in the context of this invention refers to the intentional or unintentional application of an API in a dose that is higher than the dose prescribed, recommended by a skilled person, known to be safe or known not to exert a primarily harmful effect. A harmful effect of API misuse can be the development of addictions and physical or psychological damages including death. The term misuse can also be used in the context of an API for which there is no dose prescribed, recommended by a skilled person, known to be safe or known not to exert a primarily harmful effect. In this case the misuse dose can be a harmful dose, in particular a lethal dose. Misuse can also be abuse, for example when a dose of an API is applied for which no safe dose is known for pleasure. Misuse can also be overdosing, for example when a dose of an API is applied that is higher than a dose prescribed, recommended by a skilled person, known to be safe or known not to exert a primarily harmful effect.

The term "overdosing" as used in the context of this invention refers to the application of an API or a substance in a dose that is higher than the dose prescribed, recommended by a skilled person, known to be safe or known not to exert a primarily harmful effect. A harmful effect of overdosing can be the development of addictions and physical or psychological damages including death. Overdosing can refer to the intentional application of a lethal dose of an API or substance in order to induce death. Overdosing can also refer to the administration of a substance for which no therapeutic effect, therapeutic index or therapeutic range is known. Overdosing can be abuse, for example when a substance is applied in a lethal dose for pleasure. Overdosing can also be misuse, for example when an API is applied in a dose higher than a dose prescribed, recommended by a skilled person, known to be safe or known not to exert a primarily harmful effect.

The term "therapeutic dose" as used in the present invention refers to the dosing range of a selected API in a selected indication for which a therapeutic effect is achieved according to a risk-benefit balance. The therapeutic dose of a selected API can be inferred from the therapeutic index of the respective API. The therapeutic dose of a selected API can also be determined from the therapeutic range of the respective API. A therapeutic dose can refer to the dosing range from the minimum effective dose and up to the minimum toxic dose. A therapeutic dose can be a primary therapeutic dose. A primary therapeutic dose can refer to the therapeutic dose of a selected API in a primary indication. A therapeutic dose can exert a safe and efficacious primary therapeutic effect.

The term "subtherapeutic" in the context of this invention relates to a dose of an API that falls below and/or remains below a therapeutic dose for the specific API. The term subtherapeutic can also refer to the plasma concentration of an API that falls below and/or remains below the therapeutic plasma concentration of the specific API as consequence of a dose of the API applied. In cases in which a therapeutic dose and/or a therapeutic plasma concentration are not know, subtherapeutic can refer to a dose and/or a plasma concentration that falls below and/or remains below a concentration range for which the skilled person expects a therapeutic effect. A subtherapeutic dose can refer to a single dose or a dosing regimen of an API. A subtherapeutic dose can refer to the primary therapeutic effect and be referred to as primary subtherapeutic dose. At a subtherapeutic dose different effects can occur, for example at a subtherapeutic dose no relevant primary therapeutic effect is achieved, a therapeutic effect for a different or secondary indication can be achieved and/or a different, not necessarily therapeutic effect is achieved.

In an embodiment, a therapeutic anesthetic dose of propofol for onset of sedation is 1.5 - 2.5 mg/kg body weight propofol. A therapeutic anesthetic dose of propofol for onset of sedation is 1.5 mg/kg body weight in a full-grown human subject and a toxic effect can occur at more than 1.5 mg/kg body weight. A therapeutic anesthetic dose of propofol for onset of sedation is 2.5 mg/kg body weight in a not full-grown human subject and a toxic effect can occur at more than 2.5 mg/kg body weight. In a different embodiment, a therapeutic sedative systemic level of propofol is about 1.5 - 2.5 $\mu$g/ml blood with up to 4 $\mu$g/ml blood for deep sedation or hypnosis. In this embodiment a toxic effect can occur at a systemic concentration of more than 4 $\mu$g/ml propofol. In a different embodiment, a therapeutic anesthetic dose of propofol can also be not more than 4mg/kg body weight per h. Therefore, a primary therapeutic dose of propofol can be 1.5 mg/kg body weight. In this embodiment, the primary indication is the use in anesthesia and a subtherapeutic dose is a dose below 1.5 mg/kg body weight for a secondary therapeutic effect.

In an embodiment, the dosage unit according to the invention can be used to prevent that a primary therapeutic effect of the selected API can be achieved by limiting the maximum applicable dose to a subtherapeutic dose for the primary indication. In this embodiment, a primary subtherapeutic dose can enable a therapeutic effect for a different or secondary indication and enable a secondary therapeutic effect. In this embodiment, the primary therapeutic effect can be an anesthetic effect if the API comprised in the dosage unit was selected as anesthetic and/or the primary effect can be an analgesic effect if the API comprised in the dosage unit was selected as analgesic. In this embodiment, the dose of a selected API is selected in such a way that in case of covering all non-keratinized parts of the mouth of a subject by multiple said dosage units, a primary therapeutic effect is prevented, the total dose of API applied is restricted to remain below the minimum dose for the primary therapeutic effect. Preferred combinations of dosage and area of the dosage units can be found in Tab. 1 - 4. In this embodiment, the target combined API dose as stated in Tab. 1 - 4 refers to the maximum dose for a secondary therapeutic effect.

The term "primary therapeutic effect" as used in this invention refers to a combination of API, indication and optionally dose of the API. A primary therapeutic effect used in the present invention can refer to the therapeutic effect in a primary indication for which a selected API is to be applied. In this context the "primary indication" can be understood as the main or principal indication. An indication can be a disease or a purpose. If a selected API is commonly applied in a certain indication, this indication can be referred to as primary indication of the selected API. The effect the selected API has in this primary indication can be referred to as primary therapeutic effect. A primary therapeutic effect can be achieved by application of a therapeutic dose of the selected API. Application of lower doses or subtherapeutic doses of the selected API can result in the full or optimal therapeutic effect. The presence of a primary indication can imply that there are further indications in which the API can have a therapeutic effect. Such further indications can be known or unknown to the skilled person. For example, the primary indication of propofol is the use in anesthesia. In humans, a sedative effect can be achieved if propofol is present in the blood at levels of 1.5 - 2.0 $\mu$g/ml, a hypnotic effect of propofol can be achieved if propofol is systemically present at levels of not more than 4.05 $\pm$ 1.01 $\mu$g/ml. In this embodiment, to prevent a toxic effect. A primary therapeutic effect of propofol as anesthetic, can be achieved at systemic levels of 1.5 - 2.0 $\mu$g/ml. Accordingly, for inducing the onset of sedation a minimum dose of 1.5 mg/kg body weight propofol has to be applied. Therefore, a secondary therapeutic effect of propofol can be achieved at systemic levels of up to 1.5 $\mu$g/ml. Alternatively, a secondary therapeutic effect of propofol can be achieved at less than 1.5 mg/kg body weight.

In an embodiment, a primary therapeutic effect can also refer to the therapeutic effect of a selected API in an indication in which the API is commonly applied. In this embodiment, the primary therapy can be the first treatment given for a disease and referred to as first-line therapy or induction therapy. This can be the case if for a given indication a certain treatment is commonly applied first. The API administered can be a part in a combination treatment, such as surgery and medication or a combination of more than one API. An example of this embodiment can be first-line therapy in cancer.

The term "active pharmaceutical ingredient" (API) refers to a compound which exerts a pharmacological effect in a subject. An API is thus intended to furnish pharmacological activity or to otherwise have an effect in the diagnosis, cure, mitigation, treatment or prevention of disease, or to have effect in restoring, correcting or modifying physiological functions in a subject. Specifically, the API can be a small molecule, a macromolecule, a protein, peptide, peptide fragment, nucleic acid, nucleotide fragment, gene, aptamer, or any combination of the above. Advantegeously, the API selected for a dosage unit are suitable for oral uptake and/or transmucosal delivery in particular over the non-keratinized mucosa of the orval cavity. Such API are known to the skilled person.

In a preferred embodiment of the invention, the formulation of the film consists of up to 25% (w/w) API, even more preferred of up to 22.5% (w/w) API and most preferred of up to 20% (w/w) API.

In an embodiment of the invention, the API are selected from the group of hormones, particular proteohormones, metabolic modulators, growth factors, endogenous peptide analogues, psychiatric medication, anabolic agents, beta2 agonists, hallucinogens, muscle relaxants, analgesics, anxiolytics, sedatives, hypnotics, narcotics and/or anesthetics. In a preferred embodiment, an analgesic, anxiolytic, sedative, hypnotic and/or anesthetic is selected, even more preferably an analgesic, anxiolytic, sedative, hypnotic, narcotics and/or anesthetic from the group of triptans, ditans such as lasmiditan, NSAIDs, glucocorticoid steroids, salicylates and derivatives, phenylacetic acid derivatives, 2-phenylpropionic acid derivatives, 4-aminophenol derivatives, pyrazolones, selective COX2 inhibitors, anti-depressants, anti-convulsant drugs, opioids, muscle relaxants, barbiturates, benzodiazepines, etomidates, ketamine, propofol, anti-histamines or local anesthetics is selected.

In a different embodiment of the invention the dosage unit are for use in the treatment of a metabolic disorder in a subject in the need thereof, preferably a human subject, characterized in that a hormone, particularly a proteohormone, is selected and the metabolic disorder is selected from the groups of acid-base imbalances, metabolic brain diseases, disorders of calcium metabolism, DNA repair-deficiency disorder, glucose metabolism disorders, hyperlactatemia, iron metabolism disorders, lipid metabolism disorders, malabsorption syndromes, metabolic syndrome X, inborn error of metabolism, mitochondrial diseases, phosphorus metabolism disorders, porphyrias, proteostasis, preferably a dosage unit comprising a proteohormone, particularly insulin is selected for use in the treatment of a glucose metabolism disorder, in particular diabetes.

In a different embodiment of the invention the dosage unit are for use in the treatment of a nervous system disease in a subject in the need thereof, preferably a human subject, characterized in that an analgesic and/or anesthetic is selected and the nervous system disorder is selected from the group of addiction, arachnoid cysts, attention deficit/hyperactivity disorder (ADHD), autism, bipolar disorder, catalepsy, depression, encephalitis, epilepsy/seizures, infection, locked-in-syndrome, meningitis, migraine, multiple sclerosis, myelopathy, neurophatic pain Tourette's and neurodegenerative disorders including Alzheimer's, Huntington's disease and Parkinson's, preferably the API is selected as anesthetic and the disease is selected as migraine, more preferably the API is selected as propofol and the disease is selected as migraine, most preferably the API is selected as propofol in a dose of less than 5 mg, the area of the dosage unit is selected as 6 cm$^2$ and the disease is selected as migraine.

The term "area weight" as used in the context of this invention can refer to the weight of the dosage unit or a film has in relation to its area. Typically the area weight is provided in g/m$^2$. The area weight is a relevant quality attribute of a dosage unit of a film, since it defines the API content of the prepared film. The area weight of a film can for example be adjusted by defining the gap height of the coating knife during solvent casting. After the matrix comprising the API was deposited on a substrate, the area weight can be adjusted for example by reducing the gap height of the coating knife during manufacture. This step can be repeated until the desired area weight of the film is met. Preferred area weights of a film and/or dosage unit are in the range of 5 - 500 g/m$^2$, preferably 10 - 400 g/m$^2$, further preferably 20 - 350 g/m$^2$, further preferably 30 - 300 g/m$^2$, further preferably 40 - 250 g/m$^2$, further preferably 50 - 200 g/m$^2$, further preferably 60 - 150 g/m$^2$, further preferably 70 - 130 g/m$^2$, further preferably 80 - 120 g/m$^2$, further preferably 90 - 110 g/m$^2$, further preferably 100 g/m$^2$. In particular in an embodiment in which the API is selected as propofol, an area weight of 70 - 130 g/m$^2$, preferably 80 - 120 g/m$^2$, further preferably 90 - 110 g/m$^2$, further preferably 100 g/m$^2$, is preferred for the dosage unit.

In a preferred embodiment, propofol is selected as anesthetic, an area weight of 70 - 130 g/m$^2$, preferably 80 - 120 g/m$^2$, further preferably 90 - 110 g/m$^2$, further preferably 100 g/m$^2$, is selected, a dose of less than 5 mg dosage unit is selected and an area of 6 cm$^2$ is selected.

In a preferred embodiment of the invention the API is an analgesic and/or an anesthetic and an area weight of 70- 130 g/m$^2$, preferably 80 - 120 g/m$^2$, further preferably 90 - 110 g/m$^2$, further preferably 100 g/m$^2$, is selected. In a further preferred embodiment the API is a combination of at least one analgesic and one anesthetic and an area weight of 70 - 130 g/m$^2$, preferably 80 - 120 g/m$^2$, further preferably 90 - 110 g/m$^2$, further preferably 100 g/m$^2$, is selected.

In an embodiment, the API comprises at least one anesthetic, whereby the anesthetic can be selected from anxiolytics, sedatives, hypnotics or narcotics.

In an embodiment, the API comprises at least one anesthetic, whereby the anesthetic can be selected from anxiolytics, sedatives, hypnotics or narcotices administred to achieve sedation, which is reflected by a RASS score of ≤ -2.

Furthermore preferably, the anesthetic can be selected from the group consisting of anesthetics administered to achieve at least sedation, whereby sedation can be reflected by a RASS score of ≤ -2 (RASS score of ≤ -2). A sedation status as reflected by a RASS score of ≤ -2 can also be referred to as a status in which a subject is not capable of maintaining eye contact for at least 10 seconds upon being addressed. Anesthetics administered to achive sedation comprise muscle relexants, narcotics and hypnotics, including etomidate, ketamine and propofol, benzodiazepine or barbiturates. The group of benzodiazepines comprises alprazolam, bromazepam, chlordiazepoxid, clobazam, clonazepam, diazepam, chlorazepate dipotassium, flnitrazepam, flurazepam, lorazepam, lormetazepam, medazepam, midazolam, nitrazepam, oxazepam, prazepam, temazepam, tetrazepam, triazolam, zaleplon, zolpidem, zopiclon and derivatives thereof. The group of barbiturates includes barbiturate, amorbarbital, secobarbital, butabarbital, pentobarbital, phenobarbital, butalbarbital, methohexital, hexobarbital, cyclobarbital, pentobarbital and derivatives or salts thereof.

In an embodiment, the API comprises at least one analgesic, the at least one analgesic can be selected from analgesics administered in any one or more of steps 1 to 3 of the WHO pain relief ladder.

More preferably, the API can be selected from the group comprising analgesics administered in any one or more of steps 1 to 3 of the WHO pain relief ladder. Analgesics administered in any one or more of steps 1 to 3 of the WHO pain relief ladder comprise non-opioids and opioids. Analgesics that can be administered in step 1 of the WHO pain relief ladder include triptanes, ditans, such as lasmiditan, NSAIDs, glucocorticoid steroids, salicylates, phenylacetic acid, 2-phenylpropionic acid, 4-aminophenol derivatives, pyrazolones and derivatives thereof, anti-depressants and anti-convulsant drugs. Opioids that can be administered in step 2 of the WHO pain relief ladder include opioids for mild to moderate pain, exemplary opioids in this group include codeine, tramadol, propoxyphene, pentazocine, butorphanol, nalbuphine, buprenorphine, oxycodone and derivatives thereof. Opioids that can be administered in step 3 of the WHO pain relief ladder include opioids for moderate to severe pain, exemplary opioids in this group include morphine, fentanyl, methadone, pethidine, alfentanil, remifentanil, sufentanil, hydomorphone and oxycodone and derivatives thereof.

In an embodiment, the API is selected from the groups of analgesics and/or anesthetics, preferably selected to be an anesthetic. The anesthetic is further preferably selected as a sedative hypnotic. The sedative hypnotic is further selected as propofol.

As used herein, the term "analgesic" refers to an API that can be used for the treatment of acute and chronic pain. Some analgesics are also antipyretic or protect against fever (antipyretic) and/or anti-inflammatory (antiphlogistic). Among other classifications, analgesics can be classified according to the structures in the body controlled by the active substance. Analgesics can be divided into different groups depending on the level of the WHO pain relief ladder at which they are used.

The WHO recommends an original three-step procedure for medicinal pain therapy, which was originally developed in palliative treatment and pain therapy for tumor diseases (World Health Organization: Cancer pain relief. With a guide to opioid availability (2nd ed.). WHO (Geneva). 1996). This procedure starts with a drug treatment with non-opioid analgesics, possibly in combination with adjuvants, at step 1 and escalates if the efficacy is insufficient. At step 2, a weak opioid is administered, possibly in combination with non-opioid analgesics and/or adjuvants. Finally, treatment at step 3 requires the administration of a strong opioid, possibly in combination with non-opioid analgesics and/or adjuvants. An alternative to drug therapy for pain is invasive therapy, which is often used when drug therapy fails. The invasive measures are sometimes also referred to as step 4 of the procedure in pain therapy, i.e. after drug therapy. The invasive measures include e.g. peridural injections, spinal injections, peripheral local anesthesia, spinal cord stimulation or ganglion blockade. As used in the context of this invention, in particular the analgesics of the non-invasive steps 1 to 3 are included.

The term "anesthetic" as used in the context of this invention comprises API that can be used for damping of functions of the peripheral and/or central nervous system. The term "anesthetics" comprises anesthetics that have a concomitant function as analgesic and anesthetics that do not have a concomitant function as analgesic. The term can include anxiolytics, muscle relaxants, sedatives, hypnotics and narcotics. The term can therefore comprise API used for general or systemic anesthesia such as narcotics and hypnotics as well as muscle relaxants, antidepressants and neuroleptics with a sedative effect and API used for local anesthesia such as lidocaine or mepivacain. Anti-depressants can include trazodon, doxepin, trimipramin, amitriptylin, mirtazapin, mianserin, agomelatin. Neuroleptics can comprise phenothiazine, such as promethazin, thioxanthene, such as chlorprothixen, butyrophenone such as haloperidol, or prothipendyl. Dosage units according to the present invention preferentially comprise non gaseous sedatives to induce sedation, whereby sedation can be reflected by a RASS score of $\leq$ -2 (RASS score of $\leq$ -2). If an analgesic is administered at the same time as a sedative, the treatment is called analgosedation.

The sedation status of a patient can be scored by clinical diagnosis using different scales. The Richmond Agitation Sedation Scale (RASS) is a scale comprising ten steps for determining the status of sedation of a patient. A sedation status of -2 and less according to the RASS is considered an at least mild sedation. The RASS is considered the medical criterion standard. Further scales used to clinically diagnose the sedation status include the Ramsay Sedation Scale or Ramsay Score. Alternatively, the sedation status of a patient can be measured electronically in numerous ways known to the skilled person. Examples include the bispectral index (BIS) or the auditory evoked potential (AEP) index (aepEX). For example an electroencephalogram can be measured using a bispectral index (BIS) monitor or auditory evoked potentials (AEP) can be measured. The BIS monitor records electroencephalograms and an algorithm provides a numeric measure scaled from 0 to 100 of the hypnotic effect of anesthetic drugs on CNS activity. A BIS score of > 90 indicates an awake patient, 71 - 90 mild to moderate sedation, 61 - 70 deep sedation and 40 - 60 general anesthesia.

In an embodiment of the present invention the analgesic and/or anesthetic is selected from the groups of triptanes, ditans, such as lasmiditan, NSAIDs, glucocorticoid steroids, salicylates derivatives, phenylacetic acid derivatives, 2-phenylpropionic acid derivatives, 4-aminophenol derivatives, pyrazolones, selective COX2 inhibitors, anti-depressants, anti-convulsant drugs, opioids, anti-histamines, anxiolytics, local sedatives, sedatives, narcotics, hypnotics, muscle relaxants or neuroleptics. More preferably the analgesic and/or sedative is selected from the group of opioids, narcotics, hypnotics, sedatives or anxiolytics, preferably from narcotics and sedative hypnotics, most preferably as propofol.

In an embodiment, the pain is selected from the group of mild and medium pain. Mild and medium pain can be identified for example by the WHO pain ladder or by pain rating scales. The pain can be further distinguished based on its localization. The pain may be selected from the group of headaches, facial pain, tooth pain, throat pain, neck pain, thorax pain, back pain, flank pain, groin pain, stomach pain or pain in the extremities. A headache pain may be selected from primary or secondary headaches.

In an embodiment the pain is selected as headache, preferably a primary headache, further preferably a migraine, even more preferably a migraine selected from migraine without aura, migraine with aura, chronic migraine pediatric migraine, menstrual migraine, refractory migraine, intractable migraine or acute confusional migraine (ACM).

In an embodiment, the dosage unit is selected to comprise as API an analgesic and/or anesthetic selected as propofol, the dose selected as less than 5 mg, the area selected as 6 cm$^2$ for use in the treatment of a pain, whereby the pain is selected as a mild headache pain, preferably a primary headache, most preferably a migraine.

In an embodiment, the dosage unit is selected to comprise as API an analgesic and/or anesthetic selected as propofol, the dose selected as less than 5 mg, the area selected as 6 cm$^2$ and the disease treated as migraine, whereby the migraine can be selected from migraine with aura, such as acephalgic migraine, ocular migraine or retinal migraine, migraine without aura, chronic migraine, pediatric migraine, menstrual migraine, intractable migraine, refractory migraine or acute confusional migraine (ACM).

In an embodiment, the dosage unit is selected to comprise as API an analgesic and/or anesthetic selected as propofol, the dose selected as less than 5 mg, the area selected as 6 cm$^2$ and the pain treated selected as a medium headache pain, preferably primary headache, most preferably a migraine.

In general, the delivery time of the analgesic and/or anesthetic, i.e. the time from application of the dosage unit to entering into circulation of the analgesic and/or anesthetic comprised, can be affected by the time for release of the entire API from the dosage unit, the permeation of the API in the non-keratinized mucosa and the penetration of the API into systemic circulation. Limitation of the delivery time allows for a limitation of the combined API dose of multiple dosage units administered over a given time period. Therefore, the delivery time of an API comprised in an API according to the invention can be longer, essentially equal or shorter to the residence time.

The term "residence time" as used in the context of this invention refers to the time the dosage unit is in contact with the mucosa of the subject. The residence time can be selected to limit the delivery time of the dose of the selected API, in particular to limit the delivery time of the dose of the API by uniform, continuous delivery. The residence time can be selected to limit the therapeutic analgesic and/or anesthetic dose of the API deliverable by the dosage unit. A benefit from selecting the residence time is abuse and misuse of the API comprised in the dosage unit can be further prevented. Advantageously, if the residence time is selected accordingly, applying several dosage units one after the other has no additional effect on the therapeutic dose of the API delivered, as the delivery time of the API dose comprised can be limited to essentially equal or exceed the time for flattening of the systemic level of the API via the residence time. Regulation of the residence time can be achieved by regulation of mucoadhesion and/or disintegration time. If residence time is regulated by the time of mucoadhesion of the dosage unit, the delivery time can be shorter, essentially equal to or longer than the residence time. If residence time is regulated by disintegration of the dosage unit, the delivery time can be shorter, essentially equal to or longer than than the residence time. In an embodiment, the residence time is essentially equal to and/or longer than the delivery time.

The term "delivery time" as used in the context of this invention relates to the time that a dosage unit of a dosage form was applied via the adequate administration route until the time the API enters circulation. The delivery time can also relate to the time that the API requires from the time point that it was applied to a location until the API reaches the blood stream. In embodiments, the delivery time can include the penetration and permeation time of the API.

Moreover, residence time can be selected based on the half-life of the analgesic and/or anesthetic. In an embodiment, the residence time can be selected to be essentially equal to the half-life of the analgesic and/or anesthetic or to be longer than the half-life of the analgesic and/or anesthetic. The term "half-life" can refer to the local elimination half-life or the context-sensitive half-life or systemic half-life. Preferably the term "half-live" refers to the systemic half-life.

In an embodiment the residence time is at least 5s, 10s, 30s, 1min, 2 min, 5 min, 10 min, 30 min, 60 min, 90 min, 120 min, 150 min, 180 min, 210 min or 240 min, preferentially the residence time is at least 5s, 10s, 30s, 1min, 2 min, 5 min, 10 min, 30 min, 60 min, 90 min or 120 min, more preferentially the residence time is at least 5s, 10s, 30s, 1min, 2 min, 5 min, 10 min or 30 min.

In an embodiment the half-life of the analgesic and/or anesthetic is not more than 1 min, 5 min, 10 min, 30 min, 60 min, 90 min, 120 min, 150 min, 180 min, 210 or 240 min, preferentially not more than .1 min, 5 min, 10 min, 30 min, 60 min, 90 min or 120 min, more preferentially 1 min, 5 min, 10 min or 30 min.

If residence time is regulated by the time of mucoadhesion of the dosage unit, the dosage unit is detached from the buccal mucosa when mucoadhesion stops. If residence time is regulated by disintegration time of the dosage unit, the residence time is terminated when the dosage unit has disintegrated.

In an embodiment, in which propofol is selected as anesthetic comprised in the dosage unit, the residence time can be selected to be essentially equal to the half-life of propofol. In particular the residence is selected to be at least 1 min.

In an embodiment, a combination of selected dose of the selected analgesic and/or anesthetic, the area and the residence time prevent that an analgesic or anesthetic therapeutic dose can be exceeded over time. For example a maximum anesthetic therapeutic dose of propofol is 4mg/kg body weight per hour. Accordingly, for a subject of 70 kg the maximum therapeutic dose of propofol can be considered as 280mg per hour.

Preferred combinations of dose, area and residence time according to the present invention, when the dose of the analgesic and/or anesthetic comprised per dosage unit is selected first and the area is selected second, can be derived from the subsequent Table 3. Preferred combinations of dose, area and residence time according to the present invention, when the dose of the analgesic and/or anesthetic comprised per dosage unit is selected first and the residence time is selected second, can be derived from the subsequent Table 4.

The target combined API dose refers to the dose of the selected analgesic and/or anesthetic present over time if said dosage units are combined to cover all non-keratinized parts of a mouth. According to a preferred embodiment the non-keratinized parts of the mouth cover an area of 110 cm$^2$. The target combined API dose can be the maximal dose of an analgesic and/or a anesthetic used by the skilled person to induce an analgesic and/or anesthetic effect over a limited time, for example, over an hour.

In a preferred embodiment the area of the non-keratinized parts of the mouth of a subject, preferably a full-grown human subject, is approximately 110 cm$^2$. Likewise, this area is about 45 to 110 cm$^2$ in a not full-grown human subject. The target combined API dose is selected as 280 mg over 1h, the API is selected as propofol and the dose is selected as less than 5 mg per dosage unit and the area of the dosage unit is selected as 6 cm$^2$.

In a preferred embodiment the area of the non-keratinized parts of the mouth of a subject, preferably a human subject, is approximately 110 cm$^2$. The target combined API dose is selected as 280 mg over 1h, the API is selected as propofol and the dose is selected as less than 5 mg per dosage unit and the residence time of the dosage unit is selected as 10 min.

In a first step, a dose (d) for a dosage unit is selected and the value of the target combined API dose over time ($D_t$) is divided by (d) yielding a quotient ($x_t$). In a preferred embodiment, ($D_t$) is about 280 mg/h. In a second step the maximum product of residence time (r) and area to be covered by a single dosage unit (a) is determined by dividing the area of the non-keratinized parts of the mouth (A) by ($x_t$). In a preferred embodiment, A is about 110 cm$^2$. In a third step, an area (a) or a residence time (r) is selected as value (z). In a fourth step, the minimum residence time (r) or the minimum area to be covered (a) is determined by dividing the quotient of (A) and ($x_t$) by the selected value (z).

This calculation can be visualized as:

Step 1 (selection of d in):

$$D_t / d = x_t$$

Step 2 (determination of the product of a and r):

$$A / x_t = a * r$$

Step 3 (selection of a or r as z):

$$A / x_t = z * r \text{ or } A / x_t = a * z$$

Step 4 (determination of minimum a or r):

$$A / x_t / z = r \text{ or } A / x_t / z = a$$

With

$D_t$: value of the target combined API dose overtime t, for example per h [mg/h]
d: selected dose of the API comprised in a dosage unit [mg]
$x_t$: quotient of the target combined API dose over the time t
A: area of the non-keratinized parts of the mouth, in a preferred embodiment this is 110cm$^2$ for a human subject [cm$^2$]
a: approximate minimum area to be covered by the dosage unit for the respected selected API dose [cm$^2$]
r: residence time of a single dosage unit [h]
z: selected value for a or r

Table 3: Preferred combinations of selected dose, area covered per single dosage unit and residence time of dosage units, when the dose of the API is selected first and the area is selected second.

| Number of combination | Selected API dose per single dosage unit [mg] | Selected area of a single dosage unit [cm$^2$] | Minimum residence time [h] | Target combined API dose overtime [mg/h] |
|---|---|---|---|---|
| 201 | 20 | 10 | 0.79 | |
| 202 | | 8 | 0.98 | |
| 203 | | 6 | 1.31 | |
| 204 | | 4 | 1.96 | |
| 205 | | 2 | 3.93 | |
| 206 | 19 | 10 | 0.75 | |
| 207 | | 8 | 0.93 | |
| 208 | | 6 | 1.24 | |
| 209 | | 4 | 1.87 | |
| 210 | | 2 | 3.73 | |
| 211 | 18 | 10 | 0.71 | |
| 212 | | 8 | 0.88 | |
| 213 | | 6 | 1.18 | |
| 214 | | 4 | 1.77 | |
| 215 | | 2 | 3.54 | |
| 216 | 17 | 10 | 0.67 | |
| 217 | | 8 | 0.83 | |
| 218 | | 6 | 1.11 | |
| 219 | | 4 | 1.67 | |
| 220 | | 2 | 3.34 | |
| 221 | 16 | 10 | 0.63 | |
| 222 | | 8 | 0.79 | |
| 223 | | 6 | 1.05 | |
| 224 | | 4 | 1.57 | |
| 225 | | 2 | 3.14 | |
| 226 | 15 | 10 | 0.59 | |
| 227 | | 8 | 0.74 | |
| 228 | | 6 | 0.98 | |
| 229 | | 4 | 1.47 | |
| 230 | | 2 | 2.95 | |
| 231 | 14 | 10 | 0.55 | |
| 232 | | 8 | 0.69 | |
| 233 | | 6 | 0.92 | |
| 234 | | 4 | 1.38 | |
| 235 | | 2 | 2.75 | |

(continued)

| Number of combination | Selected API dose per single dosage unit [mg] | Selected area of a single dosage unit [cm$^2$] | Minimum residence time [h] | Target combined API dose overtime [mg/h] |
|---|---|---|---|---|
| 236 | 13 | 10 | 0.51 | |
| 237 | | 8 | 0.64 | |
| 238 | | 6 | 0.85 | |
| 239 | | 4 | 1.28 | |
| 240 | | 2 | 2.55 | |
| 241 | 12 | 10 | 0.47 | |
| 242 | | 8 | 0.59 | |
| 243 | | 6 | 0.79 | |
| 244 | | 4 | 1.18 | |
| 245 | | 2 | 2.36 | |
| 246 | 11 | 10 | 0.43 | |
| 247 | | 8 | 0.54 | |
| 248 | | 6 | 0.72 | |
| 249 | | 4 | 1.08 | |
| 250 | | 2 | 2.16 | 280 |
| 251 | 10 | 10 | 0.39 | |
| 252 | | 8 | 0.49 | |
| 253 | | 6 | 0.65 | |
| 254 | | 4 | 0.98 | |
| 255 | | 2 | 1.96 | |
| 256 | 9 | 10 | 0.35 | |
| 257 | | 8 | 0.44 | |
| 258 | | 6 | 0.59 | |
| 259 | | 4 | 0.88 | |
| 260 | | 2 | 1.77 | |
| 261 | 8 | 10 | 0.31 | |
| 262 | | 8 | 0.39 | |
| 263 | | 6 | 0.52 | |
| 264 | | 4 | 0.79 | |
| 265 | | 2 | 1.57 | |
| 266 | 7 | 10 | 0.28 | |
| 267 | | 8 | 0.34 | |
| 268 | | 6 | 0.46 | |
| 269 | | 4 | 0.69 | |
| 270 | | 2 | 1.38 | |

(continued)

| Number of combination | Selected API dose per single dosage unit [mg] | Selected area of a single dosage unit [cm$^2$] | Minimum residence time [h] | Target combined API dose overtime [mg/h] |
|---|---|---|---|---|
| 271 | 6 | 10 | 0.24 | |
| 272 | | 8 | 0.29 | |
| 273 | | 6 | 0.39 | |
| 274 | | 4 | 0.59 | |
| 275 | | 2 | 1.18 | |
| 276 | 5 | 10 | 0.20 | |
| 277 | | 8 | 0.25 | |
| 278 | | 6 | 0.33 | |
| 279 | | 4 | 0.49 | |
| 280 | | 2 | 0.98 | |
| 281 | | 10 | 0.16 | |
| 282 | 4 | 8 | 0.20 | |
| 283 | | 6 | 0.26 | |
| 284 | | 4 | 0.39 | |
| 285 | | 2 | 0.79 | |
| 286 | 3 | 10 | 0.12 | |
| 287 | | 8 | 0.15 | |
| 288 | | 6 | 0.20 | |
| 289 | | 4 | 0.29 | |
| 290 | | 2 | 0.59 | |
| 291 | 2 | 10 | 0.08 | |
| 292 | | 8 | 0.10 | |
| 293 | | 6 | 0.13 | |
| 294 | | 4 | 0.20 | |
| 295 | | 2 | 0.39 | |
| 296 | 1 | 10 | 0.04 | |
| 297 | | 8 | 0.05 | |
| 298 | | 6 | 0.07 | |
| 299 | | 4 | 0.10 | |
| 300 | | 2 | 0.20 | |

Table 4: Preferred combinations of selected dose, area covered per single dosage unit and residence time of dosage units, when the dose of the API is selected first and the residence time is selected second.

| Number of combination | Selected API dose per single dosage unit [mg] | Selected residence time [h] | Minimum area of a single dosage unit [cm²] | Target combined API dose overtime [mg/h] |
|---|---|---|---|---|
| 301 | 20 | 1/12 (5 min) | 94.3 | |
| 302 | | 1/6 (10 min) | 47.1 | |
| 303 | | 1/4 (15 min) | 31.4 | |
| 304 | | 1/3 (20 min) | 23.6 | |
| 305 | | 1/2 (30 min) | 15.7 | |
| 306 | 19 | 1/12 (5 min) | 89.6 | |
| 307 | | 1/6 (10 min) | 44.8 | |
| 308 | | 1/4 (15 min) | 29.9 | |
| 309 | | 1/3 (20 min) | 22.4 | |
| 310 | | 1/2 (30 min) | 14.9 | |
| 311 | 18 | 1/12 (5 min) | 84.9 | |
| 312 | | 1/6 (10 min) | 42.4 | |
| 313 | | 1/4 (15 min) | 28.3 | |
| 314 | | 1/3 (20 min) | 21.2 | |
| 315 | | 1/2 (30 min) | 14.1 | |
| 316 | 17 | 1/12 (5 min) | 80.1 | |
| 317 | | 1/6 (10 min) | 40.1 | |
| 318 | | 1/4 (15 min) | 26.7 | |
| 319 | | 1/3 (20 min) | 20.0 | |
| 320 | | 1/2 (30 min) | 13.4 | |
| 321 | 16 | 1/12 (5 min) | 75.4 | |
| 322 | | 1/6 (10 min) | 37.7 | |
| 323 | | 1/4 (15 min) | 25.1 | |
| 324 | | 1/3 (20 min) | 18.9 | |
| 325 | | 1/2 (30 min) | 12.6 | |
| 326 | 15 | 1/12 (5 min) | 70.7 | |
| 327 | | 1/6 (10 min) | 35.4 | |
| 328 | | 1/4 (15 min) | 23.6 | |
| 329 | | 1/3 (20 min) | 17.7 | |
| 330 | | 1/2 (30 min) | 11.8 | |
| 331 | 14 | 1/12 (5 min) | 66.0 | |
| 332 | | 1/6 (10 min) | 33.0 | |
| 333 | | 1/4 (15 min) | 22.0 | |
| 334 | | 1/3 (20 min) | 16.5 | |
| 335 | | 1/2 (30 min) | 11.0 | |

(continued)

| Number of combination | Selected API dose per single dosage unit [mg] | Selected residence time [h] | Minimum area of a single dosage unit [cm$^2$] | Target combined API dose overtime [mg/h] |
|---|---|---|---|---|
| 336 | 13 | 1/12 (5 min) | 61.3 | |
| 337 | | 1/6 (10 min) | 30.6 | |
| 338 | | 1/4 (15 min) | 20.4 | |
| 339 | | 1/3 (20 min) | 15.3 | |
| 340 | | 1/2 (30 min) | 10.2 | |
| 341 | 12 | 1/12 (5 min) | 56.6 | |
| 342 | | 1/6 (10 min) | 28.3 | |
| 343 | | 1/4 (15 min) | 18.9 | |
| 344 | | 1/3 (20 min) | 14.1 | |
| 345 | | 1/2 (30 min) | 9.4 | |
| 346 | 11 | 1/12 (5 min) | 51.9 | |
| 347 | | 1/6 (10 min) | 25.9 | |
| 348 | | 1/4 (15 min) | 17.3 | |
| 349 | | 1/3 (20 min) | 13.0 | |
| 350 | | 1/2 (30 min) | 8.6 | 280 |
| 351 | 10 | 1/12 (5 min) | 47.1 | |
| 352 | | 1/6 (10 min) | 23.6 | |
| 353 | | 1/4 (15 min) | 15.7 | |
| 354 | | 1/3 (20 min) | 11.8 | |
| 355 | | 1/2 (30 min) | 7.9 | |
| 356 | 9 | 1/12 (5 min) | 42.4 | |
| 357 | | 1/6 (10 min) | 21.2 | |
| 358 | | 1/4 (15 min) | 14.1 | |
| 359 | | 1/3 (20 min) | 10.6 | |
| 360 | | 1/2 (30 min) | 7.1 | |
| 361 | 8 | 1/12 (5 min) | 37.7 | |
| 362 | | 1/6 (10 min) | 18.9 | |
| 363 | | 1/4 (15 min) | 12.6 | |
| 364 | | 1/3 (20 min) | 9.4 | |
| 365 | | 1/2 (30 min) | 6.3 | |
| 366 | 7 | 1/12 (5 min) | 33.0 | |
| 367 | | 1/6 (10 min) | 16.5 | |
| 368 | | 1/4 (15 min) | 11.0 | |
| 369 | | 1/3 (20 min) | 8.3 | |
| 370 | | 1/2 (30 min) | 5.5 | |

(continued)

| Number of combination | Selected API dose per single dosage unit [mg] | Selected residence time [h] | Minimum area of a single dosage unit [cm$^2$] | Target combined API dose overtime [mg/h] |
|---|---|---|---|---|
| 371 | 6 | 1/12 (5 min) | 28.3 | |
| 372 | | 1/6 (10 min) | 14.1 | |
| 373 | | 1/4 (15 min) | 9.4 | |
| 374 | | 1/3 (20 min) | 7.1 | |
| 375 | | 1/2 (30 min) | 4.7 | |
| 376 | 5 | 1/12 (5 min) | 23.6 | |
| 377 | | 1/6 (10 min) | 11.8 | |
| 378 | | 1/4 (15 min) | 7.9 | |
| 379 | | 1/3 (20 min) | 5.9 | |
| 380 | | 1/2 (30 min) | 3.9 | |
| 381 | 4 | 1/12 (5 min) | 18.9 | |
| 382 | | 1/6 (10 min) | 9.4 | |
| 383 | | 1/4 (15 min) | 6.3 | |
| 384 | | 1/3 (20 min) | 4.7 | |
| 385 | | 1/2 (30 min) | 3.1 | |
| 386 | 3 | 1/12 (5 min) | 14.1 | |
| 387 | | 1/6 (10 min) | 7.1 | |
| 388 | | 1/4 (15 min) | 4.7 | |
| 389 | | 1/3 (20 min) | 3.5 | |
| 390 | | 1/2 (30 min) | 2.4 | |
| 391 | 2 | 1/12 (5 min) | 9.4 | |
| 392 | | 1/6 (10 min) | 4.7 | |
| 393 | | 1/4 (15 min) | 3.1 | |
| 394 | | 1/3 (20 min) | 2.4 | |
| 395 | | 1/2 (30 min) | 1.6 | |
| 396 | 1 | 1/12 (5 min) | 4.7 | |
| 397 | | 1/6 (10 min) | 2.4 | |
| 398 | | 1/4 (15 min) | 1.6 | |
| 399 | | 1/3 (20 min) | 1.2 | |
| 400 | | 1/2 (30 min) | 0.8 | |

The mucoadhesiveness of the dosage unit can stem from the ratio and/or type of polymer admixed into the unit. Alternatively it can result from the ratio and/or type of plasticizer or API comprised in the dosage unit. Alternatively it can result from the combination of ratio and/or type of polymer and plasticizer, the combination of ratio and/or type of polymer and API, the combination of ratio and/or type of plasticizer and API or the combination of ratio and/or type of polymer, plasticizer and API comprised in the dosage unit.

The term "disintegrating" as used in the context of this invention refers to the ability of a dosage unit to eventually dissolve when applied in the oral cavity, in particular when applied to the non-keratinized mucosa. Disintegration time refers to the time it takes for the dosage unit to essentially disintegrate completely. In a preferred embodiment, the disintegration time of the dosage unit can be limited. Limitation of the disintegration time can be used to limit the residence time of the dosage unit.

## Compositions for producing the film of the dosage unit

In a further embodiment, the formulation comprising a polymer or mixture of polymers, a solvent and a selected API, such as a selected analgesic and/or anesthetic is free of a plasticizer but presents a plasticizing property. In this embodiment, the plasticizing property can be derived from an adjuvant, the API or a polymer present in the formulation in particular the plasticizing property can be derived from a polymer present in the formulation. Polymers with plasticizing properties can be selected from the group comprising hydroxypropyl cellulose (HPC). Polymers of the hydroxypropyl cellulose group comprise commercially available HPC products such as Klucel EF, Klucel EX, Klucel EXF, Klucel JX and Klucel JXF (Ashland Aqualon Functional Ingredients, USA).

Preferably, the formulation of the dosage unit according to the invention comprises a solvent, a polymer and a selected API. Optionally the formulation of the dosage unit according to the invention can comprise a plasticizier. Accordingly, in a different embodiment the formulation of the dosage unit according to the invention comprises a solvent, a polymer, a plasticizer and an API. Other ingredients or additives may be added to blend for the various purposes and include, but are not limited to bulk fillers, binding agents, thickening agents, softeners, surfactants, stabilizing agents, buffering agents, emulsifiers, disintegrants, flavoring agents, sweetening agents, colorants and the like, which may provide a benefit, but are not essential for forming the dosage unit matrix or for the pharmaceutical activity of the dosage unit.

In a preferred embodiment, the composition for producing the film of the dosage unit comprises:

    a. solvent up to 95% by weight, up to 90% by weight, up to 80% by weight, up to 70% by, or up to 60% by weight; and
    b. polymer up to 35 % by weight, up to 30% by weight, up to 25%, by weight, or up to 20% by weight; and
    c. API up to 30% by weight, up to 20% by weight, up to 10% by weight, or up to 5% by weight; and
    d. optionally a plasticizer of up to 15 % by weight, up to 10% by weight or up to 5 % by weight;

whereby preferably the polymer is a hydrophilic polymer.

In a preferred embodiment, the composition for producing the film of the dosage unit comprises:

    a. solvent: 50 - 95 % by weight; preferably 60 - 85% by weight, more preferably, 60 - 75% by weight; and
    b. polymer: 1 - 35% by weight, preferably 5 - 30% by weight, more preferably, 10 - 25% by weight, and
    c. API: 0.1 - 30% by weight, preferably 1 - 20% by weight, more preferably 1-10 % by weight; and
    d. optionally plasticizer: 0.1 - 15 % by weight, preferably 1 - 10 % by weight, more preferably 2 - 6% by weight;

whereby preferably the polymer is a hydrophilic polymer.

In a further preferred embodiment, the solid content of the composition for producing the film of the dosage unit comprises

    a. polymer at least 60%, preferably at least 70% and more preferably at least 80 % (w/w) of the solid content; and
    b. plasticizer up to 30 %, preferably up to 25% and more preferably up to 20 % (w/w) of the solid content; and
    c. API up to 25%, preferably 22.5 % and more preferably up to 20 % (w/w) of the solid content

whereby preferably the polymer is a hydrophilic polymer.

In a further embodiment, a dosage unit for use in the treatment of migraine can be obtained from a composition for producing the film which comprises

    a. polymer at least 60%, preferably at least 70% and more preferably at least 80 % (w/w) of the solid content; and
    b. plasticizer up to 30 %, preferably up to 25% and more preferably up to 20 % (w/w) of the solid content; and
    c. propofol as API up to 25%, preferably 22.5 % and more preferably up to 20 % (w/w) of the solid content

whereby preferably the polymer is a hydrophilic polymer.

Preferably, the dosage unit of the invention is dissolvable in water. Then, when applied onto the mucosa, the film can dissolve. Upon such dissolution of the film the API more easily penetrates and permeates through the mucosa. The dosage unit of this embodiment is particularly suitable for transmucosal systemic delivery of the API comprised in the dosage unit. In a further preferred embodiment, the disintegration time of the dosage unit can be limited. Limitation of the disintegration time can be used to limit the residence time of the dosage unit. Limitation of the disintegration time can occur in terms of limiting the minimum disintegration time or in terms of limiting the maximum disintegration time.

Limiting the minimum disintegration time can be achieved by reducing the water solubility of the dosage unit while limiting the maximum disintegration time can be achieved by increasing the water solubility of the dosage unit.

The dosage unit preferably is formed by polymers, in particular by hydrophilic polymers. Polymers can affect the mucoadhesivness of the dosage unit, for example the type of polymer selected or the ratio of polymer in the formulation can affect the mucoadhesiveness of the dosage unit. The film in the pharmaceutical preparation of the invention thus preferably comprises at least one hydrophilic polymer. In a particularly preferred embodiment, all polymers of the film are hydrophilic polymers. A polymer can be selected from the group of chitosan and derivatives to modify the time of mucoadhesion. A polymer can be selected from the group of chitosan, PVP, polyacrylic acid and/or poloxamer to increase the residence time by increasing mucoadhesion. Advantageously, the residence time can be modified by the selection of a polymer. Preferentially chitosan is selected as polymer to increase the mucoadhesiveness and therefore the time of residence of the dosage unit.

The term "polymer" refers to the component of a film that comprises repetitive monomeric units that were polymerized and which can be molten, reshaped and cooled down to form a film. A "polymer" as used in the context of this invention can refer to a molecule that is a chain of smaller repeating units, so called monomers, formed by carbon-carbon bonds. The carbon-carbon bonds of the polymer can form long straight chains known as linear polymers, parts of the polymer can branch off from the chain, forming so called branched polymers in which the main chain can be distinguished from side chains, or two or more polymer chains can be covalently linked two- or three dimensionally by such side chain branch forming cross-linked polymers. Polymers can be characterized for example by the extent and type of branching, the crystal structure and the molecular weight.

Polymers can be distinguished into synthetic polymers made from petroleum and other hydrocarbons or naturally occurring polymers such as starch. Examples of synthetic polymers include polyethylene (PE) which is a polymer formed from monomeric ethylene molecules and which is the most common plastic. Naturally occurring polymers include starch and cellulose which are made from long linear chains of glucose monomers.

The term "hydrophilic polymer" also refers to a polymer or copolymer which contains polar or charged functional groups, rendering them soluble in water. Preferably, the polymer component of the composition used for producing the film of the dosage unit is a water soluble or hydrophilc polymer.

Hereinafter the term "polymer" collectively refers to polymers and copolymers unless otherwise explicitly mentioned.

The formulation of the dosage unit can comprise up to 15 % by weight polymer, preferably between 1 to 10 % by weight polymer.

In a preferred embodiment of the invention the formulation of the film consists of at least 60 % (w/w) hydrophilic polymer, even more preferred 70% (w/w) hydrophilic polymer and most preferred at least 80% (w/w) hydrophilic polymer.

In a preferred embodiment of the invention the hydrophilic polymer consists of at least 50% (w/w), even more preferred of at least 60% (w/w) and most preferred of at least 70% (w/w) of monomer residues which contain polar or charged groups. In a preferred embodiment, the hydrophilic polymer is selected from the group consisting of starch and derivatives thereof such as high amylase starch, cellulose and derivatives thereof, hydroxypropylated high amylase starch, dextrin, maltodextrins or pullulan, polyacrylic acid, polyvinyl pyrrolidone (PVP), polyvinyl alcohol, polyvinyl acetate, poly(vinylpyrrolidone-co-vinyl alcohol), polysaccharides, alginate, glycogen, amylopectin, pectin, chitin, callose, ethylenoxid and propylenoxid block polymers such as polyethylene glycol, and xanthan gum, tragacanth gum, guar gum, acacia gum, Arabic gum, methylmethacrylate copolymer, carboxyvinyl polymer, levan, elsinan, collagen, zein, gluten, soy protein isolate, whey protein isolate, casein and mixtures thereof. More preferably the polymer is selected from pullulan, polyvinyl pyrrolidone (PVP), polysaccharides, high amylose starch, amylopectin, pectin, chitin, callose, cellulose and derivatives thereof (such as methyl cellulose (MC), hydroxymethyl cellulose (HMC), hydroxyethyl cellulose (HEC), carboxymethyl cellulose (CMC), hydroxypropylmethyl cellulose (HPMC) or hydroxypropyl cellulose (HPC)) as well as combinations of two or more polymers or copolymers thereof. Even more preferably the hydrophilic polymers are selected from the group consisting of pullulan, polyvinyl pyrrolidone (PVP), polyvinyl alcohol, polysaccharides and cellulose (including derivatives as stated above) or mixtures thereof. Even more preferred is the group consisting of pullulan, polyvinyl pyrrolidone (PVP), polyvinyl alcohol, cellulose and derivatives thereof (such as methyl cellulose (MC), hydroxymethyl cellulose (HMC), hydroxyethyl cellulose (HEC), carboxymethyl cellulose (CMC), hydroxypropylmethyl cellulose (HPMC) or hydroxypropyl cellulose (HPC)) as well as combinations of two or more polymers or copolymers thereof. Most preferably the polymer is selected from pullulan or polyvinyl pyrrolidone (PVP).

In specifically preferred embodiments, the hydrophilic polymer is a mixture of pullulan having ratio 10 to 25 %, hydroxypropylcellulose having a ratio of 45 to 65 % and carboxymethylcellulose having a ratio of 20 to 40%. The ratios of polymers refer to the relative amounts in relation to the total amount of polymer in the mixture. Thus, in a polymer mixture comprising 10 % pullulan, 10 % of all polymers comprised in the formulation are pullulan.

In a further specifically preferred embodiment, the hydrophilic polymer is a mixture of two variants of polyvinyl pyrrolidione (PVP), Kollidon 90F and Kollidon VA64, whereby Kollidon 90F has a ratio of 65 to 90% and Kollidon VA64 has a ratio of 10 to 35 % of the the total amount of polymer in the mixture.

In a further specifically preferred embodiment, the hydrophilic polymer is a mixture of polyvinyl alcohol and a polyvinyl pyrrolidone, wherein the polyvinyl alcohol has a ratio of 45 to 60 % and the polyvinyl pyrrolidone has a ratio of 40 to 55 % the total amount of polymer in the mixture.

The term "solvent" in the context of this invention refers to the substance that dissolves the ingredients of the formulation forming the dosage unit. The solvent preferably is a polar solvent. More preferably the polar solvent is selected from water, alkyl alcohols in particular ethanol, acids, in particular HCl and bases, in particular NaOH. The formulation of the dosage unit can comprise up to of up to 95% by weight solvent, 1 - 90% by weight solvent, 1 - 80% by weight solvent, 1 - 70% by weight solvent, 1 - 60% by weight solvent.

A "plasticizer" as used in the context of this invention is an agent that increases the plasticity and the flexibility of a material. A "plasticizer" is an agent that increases the flexibility, plasticity or fluidity of the oral film. A plasticizer is an agent that alters the physical properties of the dosage unit. A plasticizer decreases the attraction between polymer chains to make them more flexible. Two main principals of plasticization can be distinguished: internal plasticization and external plasticization. A polymer can be internally plasticized by chemically modifying the polymer or monomer so that the flexibility is increased. In this principal, the plasticizer is copolymerized with the polymer and becomes an integral part of the polymer chain. On the other hand external plasticizers are low volatile substances that are added to polymers and which interact with the polymer chains but are not chemically attached to them by primary bonds. Moreover, plasticizers can be separated into primary and secondary plasticizers or into water soluble and water insobluble.

A plasticizer can affect the mucoadhesiveness of the dosage unit, for example the type plasticizer or the ratio of plasticizer in the formulation of the dosage unit can affect the mucoadhesivness of the dosage unit. A plasticizer according to the invention is in particular selected from the group comprising glycerol, polyethylene glycol, propylene glycol, triethyl citrate sorbitol, dylitol, sylitol, 1,3-butandiole, isopropylpalmitate, dibutylsebacate, paraffin oil and combination thereof. The plasticizer for use in a composition for producing the film of the dosage unit preferably are hydrophilic and/or water soluble. A preferred example of plasticizer is glycerol. The formulation of the dosage unit can comprise up to 15 % by weight plasticizer, preferably between 1 to 10 % by weight plasticizer. Preferably the plasticizer is selected from glycerol and polyethylene glycol, more preferably from glycerol and low molecular polyethylene glycol. Most preferably the plasticizer is selected from glycerol and polyethylene glycol 400 (PEG 400).

In a preferred embodiment of the invention the formulation of the film consists of up to 30% (w/w) plasticizer, even more preferred of up to 25% (w/w) plasticizer and most preferred of up to 20% (w/w) plasticizer.

A "sweetening agent" as used in the context of this invention is an artificial or natural sweetener, an agent providing a taste of sweetness to the dosage unit. Sweetening agents include sucralose, aspartame, neotame, alitame, saccharin and natural sugars. A sweetening agent can be added overcome a bitter, nauseating or unpleasant taste of a dosage unit. Preferably, the formulation of a dosage unit according to the present invention does not comprise a sweetening agent. Advantageously, the absence of sweetening agents reduces and/or prevents the misue and abuse of said dosage units.

A "flavoring agent" as used in the context of this invention is an artificial or natural flavor, an agent providing a different taste for example a fruit, mint, licorice, salty, sour, bitter, umami or sweet taste to the dosage unit. A flavoring agent can be added overcome a bitter, nauseating or unpleasant taste of a dosage unit. A flavoring agent can also be a taste masking agent, whereby the term "taste masking" agent is an agent altering the taste of the dosage unit. A taste masking agent can provide a new taste, alter the perception of the taste or can block a taste. The taste masking agents include effervescent agents including sodium bicarbonate or citric, dimenhydrinate, sodium chloride or bitter blocking agents such as adenosine monophosphate, lipoproteins, or phospholipids. Bitter blocking agents can compete with the bitter active to bind to the G-protein coupled receptors on the tongue, the receptor sites that perceive the bitter taste, thus suppressing the bitter taste. Preferably, the formulation of a dosage unit according to the present invention does not comprise a flavoring agent. Advantageously, the absence of flavoring agents reduces and/or prevents the misue and abuse of said dosage units.

A "colorant" as used in the context of this invention is an agent affecting the color of the dosage unit. A colorant includes pigments, such as titanium dioxide, or FDandC colors, EU colors, natural colors and custom pantone-matched colors.

Preferably, the dosage unit of the invention has organoleptic characteristics that do not mask the taste of the dosage unit. Advantageously, a dosage unit that has organoleptic characteristics that do not mask the taste of the dosage unit reduces and/or prevents the misuse and abuse of the respective dosage unit. Such organoleptic characteristics can result from the absence of sweetening agents in the formulation, from the absence of flavoring agents in the formulation or from absence of sweetening and flavoring agents in the formulations. More preferably, the formulation of a dosage unit according to the present invention is essentially free of flavoring agents and sweetening agent.

**BRIEF DESCRIPTION OF THE DRAWINGS**

FIG. 1    Cumulative permeation data of formulation F6 compared to a satuarated solution of propofol in an ex-vivo permetation test on porcine buccal mucosa in vertical Franz-cells.

FIG. 2    Flux data of formulation F6 compared to a satuarated solution of propofol in an ex-vivo permetation test on porcine buccal mucosa in vertical Franz-cells.

**Specific embodiments**

1. A dosage unit of a mucoadhesive oral film for use in the treatment of a disease in a subject, preferably a human subject, comprising an active pharmaceutical ingredient (API) characterized in that the dose of said API is selected in such a way that in case of covering all non-keratinized parts of the mouth of said subject by multiple said dosage units, a toxic effect of said API is prevented.

2. The dosage unit according to embodiment 1 characterized in that the subject is selected from the group of full-grown human subjects and not full-grown human subjects, whereby

    a. in full-grown human subjects the non-keratinized parts of the mouth have an area of about 150 cm$^2$, about 130 cm$^2$, about 110 cm$^2$; or

    b. in not full-grown human subjects the non-keratinized parts of the mouth have an area of up to 150 cm$^2$, up to 130 cm$^2$, up to 110 cm$^2$, preferably an area of 45 - 150 cm$^2$, of 45 -130 cm$^2$, of 45-110 cm$^2$.

3. The dosage unit according to any one or more of embodiment 1 and 2 characterized in that the API is selected from the group of hormones, particular proteohormones, metabolic modulators, growth factors, endogenous peptide analogues, psychiatric medication, anabolic agents, beta2 agonists, hallucinogens, muscle relaxants, analgesics, anxiolytics, sedatives, hypnotics and/or anesthetics.

4. The dosage unit according to any one or more of claim 1 to 3 characterized in that an analgesic, anxiolytic, sedative, hypnotic and/or anesthetic is selected from the group of triptans, ditans such as lasmiditan, NSAIDs, glucocorticoid steroids, salicylates and derivatives, phenylacetic acid derivatives, 2-phenylpropionic acid derivatives, 4-aminophenol derivatives, pyrazolones, selective COX2 inhibitors, anti-depressants, anticonvulsant drugs, opioids, muscle relaxants, barbiturates, benzodiazepines, etomidates, ketamine, propofol, anti-histamines or local anesthetics.

5. The dosage unit according to embodiment 3 for use in the treatment of a metabolic disorder in a subject in the need thereof, preferably a human subject, characterized in that a hormone, particularly a proteohormone, is selected and the metabolic disorder is selected from the groups of acid-base imbalances, metabolic brain diseases, disorders of calcium metabolism, DNA repair-deficiency disorder, glucose metabolism disorders, hyperlactatemia, iron metabolism disorders, lipid metabolism disorders, malabsorption syndromes, metabolic syndrome X, inborn error of metabolism, mitochondrial diseases, phosphorus metabolism disorders, porphyrias, proteostasis deficiencies, metabolic skin diseases, wasting syndrome and water-electrolyte imbalances.

6. The dosage unit according to embodiment 5 characterized in that the API is a proteohormon, particularly insulin, and the disease is selected as a glucose metabolism disorder, in particular diabetes.

7. The dosage unit according to any one or more of embodiment 1 to 4 for use in the treatment of a nervous system disease in a subject in the need thereof, preferably a human subject.

8. The dosage unit according to embodiment 7 characterized in that the nervous system disease is a primary headache, preferably a migraine.

9. The dosage unit according to any one or more of embodiment 7 to 8 characterized in that the primary headache is selected as migraine selected from migraine without aura, migraine with aura, migraine with aura without headache, migraine with aura with headache, migraine with brainstem aura, hemiplegic migraine, retinal migraine, chronic migraine, pediatric migraine, menstrual migraine, refractory migraine, intractable migraine or acute confusional migraine (ACM).

10. The dosage unit according to any one or more of embodiment 1 to 9 characterized in that the dosage unit has an area selected from the ranges of at least 1 cm$^2$, at least 2 cm$^2$, at least 3 cm$^2$, at least 4 cm$^2$, at least 5 cm$^2$, at least 6 cm$^2$, at least 7cm$^2$, at least 8 cm$^2$, at least 9 cm$^2$, at least 10 cm$^2$, at least 11 cm$^2$ or at least 12 cm$^2$, preferably at least 6 cm$^2$.

11. The dosage unit according to any one or more of embodiment 1 to 10 characterized in that the dose of the API is selected as a subtherapeutic dose, preferably selected as not more than 1/10, more preferably not more than 1/35, of a therapeutic dose.

12. The dosage unit according to any one or more of embodiment 1 to 11 characterized in that the dosage unit has a residence time, wherein the residence time is selected to be at least as long as the systemic half-life of the API.

13. The dosage unit according to any one or more of embodiment 1 to 12 characterized in that the organoleptic features of the composition for producing the dosage unit do not mask the taste of the dosage unit.

15. The dosage unit according to any one or more of embodiment 1 to 13 characterized in that the composition for producing the dosage unit is essentially free of flavoring agents and/or sweetening agents.

16. The dosage unit according to any one or more of embodiment 1 to 14 characterized in that the composition for producing the film of the dosage unit comprises

    a. solvent up to 95% by weight, up to 90% by weight, up to 80% by weight, up to 70% by, or up to 60% by weight; and
    b. polymer up to 35 % by weight, up to 30% by weight, up to 25% by weight, or up to 25% by weight; and
    c. API up to 30% by weight, up to 20% by weight, up to 10% by weight, or up to 5% by weight; and
    d. optionally a plasticizer of up to 15 % by weight, up to 10% by weight or up to 5 % by weight;

whereby preferably the polymer is a hydrophilic polymer.

17. The dosage unit according to any one or more of embodiment 1 to 15 for use in the treatment of migraine characterized in that the composition for producing the film of the dosage unit comprises

    a. polymer at least 60%, preferably at least 70% and more preferably at least 80 % (w/w) of the solid content; and
    b. plasticizer up to 30 %, preferably up to 25% and more preferably up to 20 % (w/w) of the solid content; and
    c. propofol as API up to 25%, preferably 22.5 % and more preferably up to 20 % (w/w) of the solid content

whereby preferably the polymer is a hydrophilic polymer.

18. The dosage unit according to any one or more of embodiment 1 to 16 for use in the treatment of migraine characterized in that the API is selected as propofol, the dosage is selected as not more than 1/10 of a therapeutic sedative dose, residence time is at least 1 min, the area of the dosage unit is at least 6 cm$^2$ and the composition for producing the dosage unit is essentially free of flavoring agents and sweetening agents.

**EXAMPLES**

**1. Dosage unit comprising propofol** - **selection of target values for area, dose and residence time**

The aim was to manufacture a dosage unit containing the active pharmaceutical ingredient propofol. The buccal film is supposed to deliver the API through the mucosal tissue into the blood stream without passing the gastro-intestinal tract because of the very low oral bioavailability of propofol. A very low propofol dose, compared to the sedative dose that is applied via i.v. injection, can be included in the dosage unit. According dosage unit can be used in the treatment of acute migraine attacks since the propofol is absorbed directly into the blood stream through the buccal mucosa. The residence time of the film at the buccal mucosa as a combination of the disintegration time of the film and the mucoadhesion of the film on the mucosa is selected in the range between 1 to 5 min to deliver the API through the mucosa. Further targeted features for the mucoadhesive, dissolvable buccal propofol film are listed in Table 5. The dosage strength of the target dosage unit was selected for a full-grown human subject with a total area of non-keratinized parts of the mouth of about 110 cm$^2$ to be about 5 mg and the target combined propofol dose not to be exceeded was selected as 100 mg. According to Tab. 1 (combination number 96) the minimum area to be covered by a dosage unit is 5.5 cm$^2$. Consequently an area of 6 cm$^2$ per single dosage unit was selected. Therefore, if a multitude of said dosage units was to cover all non-keratinized parts of the mouts of said full-grown subject[2], the combined API dose would be about 91.7 mg, since 5 mg (per dosage unit) * 110 cm$^2$ (non-keratinized area of the mouth) : 6 cm$^2$ (area per dosage unit) = 91.7 mg.

Table 5: Target attributes for a dosage unit of a propofol film

| Features | Target |
| --- | --- |
| API | Propofol |
| Dosage form | Oromucosal, mucoadhesive dissolvable film |
| Route of administration | Mucosal (buccal, sublingual...) |
| Indication | Acute migraine attack |
| Dosage strength per area | 5 mg/6 cm$^2$ film |
| Residence time | 10 s - 5 min |
| Organoleptic characteristics | No taste masking to prevent possible abuse and misuse |
| Pharmacokinetics | Immediate release via the mucosa |

A dosage unit comprising propofol in a dosage wherein the simultaneous application of multiple dosage units in the non-keratinized parts of the mouth does not exceed a sedative therapeutic dose for the treatment of acute migraine and the identification of subjects amendable for treatment with according dosage unit and treatment of subjects suffering from a migraine attack with according dosage unit would provide an innovation. Moreover, the drug approval for drug products with an anesthetic drug such as propofol for the use in non-clinical environments expects a possible prevention strategy to avoid abuse and misuse of the product if less restricted available to the public. The implementation of propofol into a mucoadhesive orally disintegrating film enables an abuse and misuse-deterrent application of propofol for acute migraine attacks due to the limited dose and limited application area at the oral mucosa. Further advantageous with regard to propofol is the fact that every part of the API dose that is taken orally after swallowing is destroyed due to the very low oral bioavailability and no therapeutic effect is experienced.

In the following section the feasibility formulation trials will be described including their composition, their handling and visual properties and for some formulations furthermore the determined content of propofol per 6 cm$^2$ film. One formulation was further evaluated in an ex-vivo permeation study on porcine buccal mucosa.

**2. Initial formulation development of propofol dosage unit**

Summary of formulations for a buccal dosage unit comprising propofol

Table 6: Formulation compositions for dosage units

| Ingredient | Formulations | | | | | | |
|---|---|---|---|---|---|---|---|
| | F1 | F2 | F3 | F4 | F5 | F6 | F7 |
| | [%] | [%] | [%] | [%] | [%] | [%] | [%] |
| *Solid content [%] (w/w)* | *23.66* | *8.8* | *13.24* | *17.2* | *15.0* | *17.2* | *32* |
| | | | | | | + 18% API surplus | |
| Pullulan | 16.4 | 63.7 | 2.2 | 12.8 | 12.7 | 12.8 | |
| PVP (Kollidon 90F) | 52.3 | | | | | | 59.3 |
| PVP (Kollidon VA64) | 13.2 | | | | | | 14.8 |
| HPMC (Pharmacoat603) | | | 60.0 | | | | |
| Chitosan | | | 11.1 | | | | |
| HPC (Klucel EF) | | | | 53.1 | 52.8 | 53.1 | |
| CMC (Blanose 7LP EP) | | | | 25.8 | 26.3 | 25.8 | |
| Polyacrylic acid (Carbopol971PNF) | | 4.3 | | | | | |
| Poloxamer (Kolliphor P407) | | 16.9 | | | | | |
| | | | | | | | |
| Glycerol | 8.3 | | 14.6 | | | | |
| PEG 400 | | | | | | | 17.6 |
| | | | | | | | |
| Propofol | 9.8 | 15.1 | 12.1 | 8.3 | 8.2 | 8.3 | 8.3 |
| | | | | | | | |
| Water | 100% | 99.04% | | 100% | 80% | 100% | 18% |
| Ethanol | | | | | 20% | | 82% |
| HCl | | | 100% | | | | |
| NaOH | | 0.96% | | | | | |
| | | | | | | | |
| Dosage: API content [mg] per 6 cm² dosage unit | n.d. | n.d. | 2.98 | 4.25 | 3.22 | 5.05 ± 0.05 | 3.65 |
| Area weight [g/m²] | ~ 70 | | ~ 100 | | | | |

The formulations shown in Tab. 6 were prepared in this feasibility study for a buccal propofol film. In general, the solvent was filled into the mixing container and the polymers and plasticizer, if used any, were added while stirring the mass. The API propofol was added when the polymer mass was fully dissolved and showed a homogenous appearance. It was noteworthy that almost every polymer mass became white after adding the propofol into the mass, probably due to emulsion formation of the lipophilic API and the hydrophilic polymer mass. The exception was formulation F7 for which the polymer mass stayed clear after adding the propofol which suggests that the propofol is better incorporated in this mass than in the other formulations.

The films were prepared by solvent casting as soon as the API was evenly stirred into the mass, forming a matrix. The solvent casting process includes the coating of the polymer mass onto a substrate, such as a PET release liner, with a coating knife. A defined gap height is adjusted at the coating knife, which results in the wet film thickness and after drying in the area weight of the prepared film. The film manufacturing including the adjustment of the gap height is repeated until the desired area weight of the film is met. The area weight is a relevant quality attribute since it defines the API content of the prepared films.

After the coating step, the wet laminate (matrix) is dried in an oven between 65 and 90 °C for 20 to 30 min depending on the solid content and the amount of solvent that needs to be evaporated. The white appearance of the wet film changed to an opaque appearance during the drying step resulting in slight milky films. The dry films were punched into 6 cm$^2$ pieces that were then evaluated regarding their handling and visual properties. Some of the prepared films were furthermore analyzed for their API content. The mucoadhesion of the buccal films that is needed to provide the buccal absorption of the API was ensured by using at least one mucoadhesive polymer in the formulations.

A formulation comprising chitosan such as formulation F3 showed reduced mucoadhesive properties. Furthermore, the chitosan is only soluble in an acidic solvent and therefore hydrochloric acid was used for film preparation in formulations containing this polymer. The films did not provide a good taste and mouthfeel since they caused a very dry mouth feel. Thereby reducing the likelihood of misuse, abuse or overdosing of according dosage units.

Alternatively, other polymers with mucoadhesive properties were challenged as ingredient for the buccal propofol dosage unit. Different Kollidon® types were used in formulation F1 and polyacrylic acid was incorporated in formulation F2 together with the addition of a Poloxamer. Polyacrylic acid showed good mucoadhesive properties.

Although all the previously named formulations provided films with sufficient handling and visual properties, another mucoadhesive polymer was tested to improve taste, mouthfeel and preparation of the formulations. The sodium carboxymethyl cellulose (CMC) provided good mucoadhesive properties and resulted in films with good handling and visual properties. They are flexible enough to be further processed and they do not stick to the liner or the packaging material.

For the HPC containing films, such as formulations F4, F5 or F6 no plasticizer was added since the polymer itself already presents some plasticizing properties. The films tend to get sticky when adding additional plasticizer.

Formulation F4 showed a propofol content below the desired 5 mg/6 cm$^2$. Therefore, in formulation F5, ethanol was added as solvent in order to prevent problems with the correct adjustment of the API content in the dried films. The film presented nice handling and visual properties but showed a low API content after the drying process. Therefore, to reach the target content of 5 mg propofol per 6 cm$^2$ dosage unit, a surplus of API was added to the matrix in formulation F6 to balance the API loss during drying. The content of 5 mg was met correctly in this case. Formulation F6 was also used as current formulation to determine the permeation capability of the propofol from a buccal film.

In formulation F7 propofol was added to formulations free of pullulan to increase the formulation variability. The films presented sufficient visual and handling properties but were not further optimized regarding the API content. Formulation F7 was the only formulation that did not turn white after adding propofol indicating a very well incorporation of the oily API into the polymer mass.

### 3. Biological example - penetration and permeation of a current formulation

The composition of the dosage unit formulation (F6) chosen for penetration and permeation experiments is presented in Table 6. The dosage units of the films were prepared with an area weight of 100,3 g/m$^2$ and a propofol content of 5,05 $\pm$ 0,05 g/6 cm$^2$ (mean $\pm$ sd).

The dosage unit prepared from formulation F6 were tested for ex-vivo permeation on porcine buccal mucosa in vertical Franz-cells. The permeation behavior of the films was compared to a saturated solution of propofol of about 167.8 $\mu$g/ml in the acceptor medium of buffer pH 7.0 (Fig. 1).No significant differences could be shown between the saturated solution and the films due to high standard deviations (Fig. 1). The dosage unit of the buccal film however indicates the trend to reach higher cumulative permeation amounts and flux rates than the saturated solution. In this first permeation trial, the steady state of the flux rate was reached after two hours whereby the flux rate of F6 reached a higher niveau (Fig. 2). However, it has to be kept in mind that the drug loading of the permeation cell (API in 1 film piece of 0.82 cm$^2$ (about 0.68 mg) and API in 1.5 ml of saturated solution (about 0.25 mg)) for the propofol comprising buccal film was higher than for the saturated solution, which might be responsible for the higher permeation.

The evaluation of the API amount that permeated from the formulation F6 film within 6 hours of the permeation study revealed that around 4 % of the API loaded to the donor compartment of the permeation cell reached the acceptor compartment. The extraction of the mucosal tissue after the permeation study with the formulation F6 film showed that around 40 % of the API loaded to the donor compartment of the permeation cell penetrated into the mucosal tissue remained in there and had not permeated into the acceptor phase yet. Thereby, successful transmucosal delivery of propofol using a dosage unit of a propofol-comprising film was shown on porcine buccal mucosa in vertical Franz-cells.

**Fig. 1: Cumulative permeation data of formulation F6 compared to a saturated solution of propofol in an ex-vivo permeation test on porcine buccal mucosa in vertical Franz-cells (n=8 for F6 and control).**

**Fig. 2: Flux data of formulation F6 compared to a saturated solution of propofol in an ex-vivo permeation test on porcine buccal mucosa in vertical Franz-cells (n=8 for F6 and control).**

**Claims**

**1.** A dosage unit of a mucoadhesive oral film for use in the treatment of a disease in a subject, preferably a human subject, comprising an active pharmaceutical ingredient (API) **characterized in that** the dose of said API is selected in such a way that in case of covering all non-keratinized parts of the mouth of said subject by multiple said dosage units, a toxic effect of said API is prevented.

**2.** The dosage unit according to claim 1 **characterized in that** the subject is selected from the group of full-grown human subjects and not full-grown human subjects, whereby

a. in full-grown human subjects the non-keratinized parts of the mouth have an area of about 150 $cm^2$, about 130 $cm^2$, about 110 $cm^2$; or

b. in not full-grown human subjects the non-keratinized parts of the mouth have an area of up to 150 cm$^2$, up to 130 cm$^2$, up to 110 cm$^2$, preferably an area of 45 - 150 cm$^2$, of 45 -130 cm$^2$, of 45 - 110 cm$^2$.

**3.** The dosage unit according to any one or more of claim 1 and 2 **characterized in that** the API is selected from the group of hormones, particular proteohormones, metabolic modulators, growth factors, endogenous peptide analogues, psychiatric medication, anabolic agents, beta2 agonists, hallucinogens, muscle relaxants, analgesics, anxiolytics, sedatives, hypnotics and/or anesthetics.

**4.** The dosage unit according to any one or more of claim 1 to 3 **characterized in that** an analgesic, anxiolytic, sedative, hypnotic and/or anesthetic is selected from the group of triptans, ditans such as lasmiditan, NSAIDs, glucocorticoid steroids, salicylates and derivatives, phenylacetic acid derivatives, 2-phenylpropionic acid derivatives, 4-aminophenol derivatives, pyrazolones, selective COX2 inhibitors, anti-depressants, anticonvulsant drugs, opioids, muscle relaxants, barbiturates, benzodiazepines, etomidates, ketamine, propofol, anti-histamines or local anesthetics.

**6.** The dosage unit according to claim 3 for use in the treatment of a metabolic disorder in a subject in the need thereof, preferably a human subject, **characterized in that** a hormone, particularly a proteohormone, is selected and the metabolic disorder is selected from the groups of acid-base imbalances, metabolic brain diseases, disorders of calcium metabolism, DNA repair-deficiency disorder, glucose metabolism disorders, hyperlactatemia, iron metabolism disorders, lipid metabolism disorders, malabsorption syndromes, metabolic syndrome X, inborn error of metabolism, mitochondrial diseases, phosphorus metabolism disorders, porphyrias, proteostasis deficiencies, metabolic skin diseases, wasting syndrome and water-electrolyte imbalances.

**7.** The dosage unit according to claim 5 **characterized in that** the API is a proteohormon, particularly insulin, and the disease is selected as a glucose metabolism disorder, in particular diabetes.

**8.** The dosage unit according to any one or more of claim 1 to 4 for use in the treatment of a nervous system disease in a subject in the need thereof, preferably a human subject.

**9.** The dosage unit according to claim 7 **characterized in that** the nervous system disease is a primary headache, preferably a migraine.

**10.** The dosage unit according to any one or more of claim 7 to 8 **characterized in that** the primary headache is selected as migraine selected from migraine without aura, migraine with aura, migraine with aura without headache, migraine with aura with headache, migraine with brainstem aura, hemiplegic migraine, retinal migraine, chronic migraine, pediatric migraine, menstrual migraine, refractory migraine, intractable migraine or acute confusional migraine (ACM).

**11.** The dosage unit according to any one or more of claim 1 to 9 **characterized in that** the dosage unit has an area selected from the ranges of at least 1 cm$^2$, at least 2 cm$^2$, at least 3 cm$^2$, at least 4 cm$^2$, at least 5 cm$^2$, at least 6 cm$^2$, at least 7cm$^2$, at least 8 cm$^2$, at least 9 cm$^2$, at least 10 cm$^2$, at least 11 cm$^2$ or at least 12 cm$^2$, preferably at least 6 cm$^2$.

**12.** The dosage unit according to any one or more of claim 1 to 10 **characterized in that** the dose of the API is selected as a subtherapeutic dose, preferably selected as not more than 1/10, more preferably not more than 1/35, of a therapeutic dose.

**13.** The dosage unit according to any one or more of claim 1 to 11 **characterized in that** the dosage unit has a residence time, wherein the residence time is selected to be at least as long as the systemic half-life of the API.

**14.** The dosage unit according to any one or more of claim 1 to 12 **characterized in that** the organoleptic features of the composition for producing the dosage unit do not mask the taste of the dosage unit.

**15.** The dosage unit according to any one or more of claim 1 to 13 **characterized in that** the composition for producing the dosage unit is essentially free of flavoring agents and/or sweetening agents.

**16.** The dosage unit according to any one or more of claim 1 to 14 **characterized in that** the composition for producing the film of the dosage unit comprises

a. solvent up to 95% by weight, up to 90% by weight, up to 80% by weight, up to 70% by, or up to 60% by weight; and
b. polymer up to 35 % by weight, up to 30% by weight, up to 25% by weight, or up to 25% by weight; and
c. API up to 30% by weight, up to 20% by weight, up to 10% by weight, or up to 5% by weight; and
d. optionally a plasticizer of up to 15 % by weight, up to 10% by weight or up to 5 % by weight;

whereby preferably the polymer is a hydrophilic polymer.

**17.** The dosage unit according to any one or more of claim 1 to 15 for use in the treatment of migraine **characterized in that** the composition for producing the film of the dosage unit comprises

a. polymer at least 60%, preferably at least 70% and more preferably at least 80 % (w/w) of the solid content; and
b. plasticizer up to 30 %, preferably up to 25% and more preferably up to 20 % (w/w) of the solid content; and
c. propofol as API up to 25%, preferably 22.5 % and more preferably up to 20 % (w/w) of the solid content

whereby preferably the polymer is a hydrophilic polymer.

**18.** The dosage unit according to any one or more of claim 1 to 16 for use in the treatment of migraine **characterized in that** the API is selected as propofol, the dosage is selected as not more than 1/10 of a therapeutic sedative dose, residence time is at least 1 min, the area of the dosage unit is at least 6 cm$^2$ and the composition for producing the dosage unit is essentially free of flavoring agents and sweetening agents.

Europäisches
Patentamt

European
Patent Office

Office européen
des brevets

# EUROPEAN SEARCH REPORT

**Application Number**

EP 20 17 5523

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/125533 A2 (BIOTA LTD [IL]; BIO GELS PHARMACEUTICALS [IS] ET AL.) 8 November 2007 (2007-11-08) * claims 1,7-9 * * page 21 - page 27; examples 1-10 * * page 12, line 24 - page 13, line 13 * | 1-4,6-17 | INV. A61K31/05 A61K9/00 A61K38/28 A61P25/06 |
| X | US 2020/016092 A1 (BERNARDO JOSE [US] ET AL) 16 January 2020 (2020-01-16) * page 1, paragraph 002 * * page 6, paragraph 58 * * page 20, paragraph 461-467 * | 1-4,6-16 | |
| X,D | US 2011/269844 A1 (LEDONNE JOHN [US]) 3 November 2011 (2011-11-03) * page 1, paragraph 6-7 * * page 2, paragraph 36 - page 3, paragraph 37 * | 1-4,8, 11-17 | |
| Y | | 18 | |
| X | NIESE SVENJA ET AL: "Development of a dosing device for individualized dosing of orodispersible warfarin films", INTERNATIONAL JOURNAL OF PHARMACEUTICS, vol. 561, 13 March 2019 (2019-03-13), pages 314-323, XP085642902, ISSN: 0378-5173, DOI: 10.1016/J.IJPHARM.2019.03.019 * page 316; table 1 * | 16 | TECHNICAL FIELDS SEARCHED (IPC) A61K A61P |
| Y | KRUSZ J C ET AL: "INTRAVENOUS PROPOFOL: UNIQUE EFFECTIVENESS IN TREATING INTRACTABLE MIGRAINE", HEADACHE, WOODBURY, NJ, UNITED STATES, vol. 40, 1 January 2000 (2000-01-01), pages 224-230, XP002948668, ISSN: 0017-8748, DOI: 10.1046/J.1526-4610.2000.00032.X * the whole document * | 18 | |

The present search report has been drawn up for all claims

| Place of search | Date of completion of the search | Examiner |
|---|---|---|
| The Hague | 12 November 2020 | Collins, Sally |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 5523

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2020

| Patent document cited in search report | | | Publication date | Patent family member(s) | | | Publication date |
|---|---|---|---|---|---|---|---|
| WO 2007125533 | A2 | | 08-11-2007 | US | 2009186107 | A1 | 23-07-2009 |
| | | | | WO | 2007125533 | A2 | 08-11-2007 |
| US 2020016092 | A1 | | 16-01-2020 | US | 2020016092 | A1 | 16-01-2020 |
| | | | | WO | 2020014431 | A1 | 16-01-2020 |
| US 2011269844 | A1 | | 03-11-2011 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011061332 A
- WO 2010062688 A1
- WO 2018091473 A1
- WO 2005055977 A2
- WO 2018208701 A1
- US 20110269844 A1

**Non-patent literature cited in the description**

- **JAESCHKE H. ACETAMINOPHEN.** Dose-Dependent Drug Hepatotoxicity and Acute Liver Failure in Patients. *Dig Dis,* 2015, vol. 33, 464-471
- **GHANEM C.I. ; PEREZ M.J. ; MANAUTOU J.E. ; MOTTINO A.D. ; MARIA J.P.** Acetaminophen from liver to brain: New insights into drug pharmacological action and toxicity. *Pharm. Res.,* 2016, vol. 109, 119-131
- *CHEMICAL ABSTRACTS,* 2078-54-8
- *Cephalalgia,* January 2018, vol. 38 (1), 1-211
- With a guide to opioid availability. Cancer pain relief. World Health Organization, 1996